# EUROPÄISCHE PATENTSCHRIFT

(11) **EP 2 229 133 B1**
(45) Veröffentlichungstag und Bekanntmachung des Hinweises auf die Patenterteilung: **19.06.2013**
(21) Anmeldenummer: 09703027.4
(22) Anmeldetag: 05.01.2009
(51) Int. Cl.: A61K 8/25, A61K 8/02, A61K 8/11, A61K 8/26, A61K 8/29, A61K 8/31, A61K 8/33, A61K 8/37, A61K 8/58, A61K 8/70, A61K 8/92, A61Q 5/06

(54) **Pulverförmige Zusammensetzungen zur Form- und Glanzgebung keratinischer Fasern**
Pulverulent compositions for the shaping and lustring of keratin fibres
Compositions pulvérulentes pour mettre en forme des fibres kératiniques et/ou leur donner du brillant

(30) Priorität: 14.01.2008 DE 102008004402
(43) Veröffentlichungstag der Anmeldung: 22.09.2010
(73) Patentinhaber: Henkel AG & Co. KGaA, 40589 Düsseldorf (DE)
(72) Erfinder: RICHTERS, Bernd, 21129 Hamburg (DE); BAYERSDÖRFER, Rolf, 22589 Hamburg (DE); HENTRICH, Dirk, 22457 Hamburg (DE)
(86) Internationale Anmeldenummer: PCT/EP2009/050055
(87) Internationale Veröffentlichungsnummer: WO 2009/090114

(56) Entgegenhaltungen:
- EP-A- 1 426 033
- WO-A-2007/051511
- US-A1- 2003 202 993
- TANAKA Y: "LIQUEFIABLE COSMETIC POWDERS" CA,, 1. Januar 1900 (1900-01-01), XP002388921
- E. Sagarin (Ed.): "Cosmetics Science and Technology", 1957, Interscience Publishers, New York, London, Sydney pages 559-563,

## Beschreibung

Die vorliegende Erfindung betrifft pulverförmige Zusammensetzungen, die maximal 20 Gew.-% Wasser und in einer flüssigen Phase 40 bis 95 Gew.% eines Öls und in einer festen Phase hydrophobierte Metalloxidere und mindestens ein film bildendes und/oder festigendes Polymer enthalten. Diese Zusammensetzung werden zur temporären Verformung keratinischer Fasern und zur Glanzgebung auf keratinischen Fasern angewendet.

Stylingmittel zur Verformung keratinischer Fasern sind lange bekannt und finden in verschiedener Ausgestaltung Einsatz zum Aufbau, zur Auffrischung und zur Fixierung von Frisuren, die sich bei vielen Haartypen nur unter Verwendung festigender Wirkstoffe erhalten lassen. Dabei spielen sowohl Haarbehandlungsmittel, die einer permanenten, als auch solche, die einer temporären Formgebung der Haare dienen, eine wichtige Rolle. Temporäre Formgebungen, die einen guten Halt ergeben sollen, ohne das gesunde Aussehen der Haare, wie zum Beispiel deren Glanz, zu beeinträchtigen, können beispielsweise durch Haarsprays, Haarwachse, Haargele, Fönwellen etc. erzielt werden.

Entsprechende Mittel zur temporären Formgebung enthalten als formgebende Komponente üblicherweise synthetische Polymere. Zubereitungen, die ein gelöstes oder dispergiertes Polymer enthalten, können mittels Treibgasen oder durch einen Pumpmechanismus auf das Haar aufgebracht werden. Haargele und Haarwachse werden hingegen in der Regel nicht direkt auf das Haar appliziert, sondern mittels eines Kamms oder der Hände im Haar verteilt.

Bekannte Formen temporärer Stylingmittel lassen sich oftmals nicht mit zufriedenstellender Genauigkeit dosieren. So lassen sich etwa Haargele, Haarcremes und Haarwachse nur noch schwierig verteilen, sobald sie einmal auf das Haar aufgebracht sind. Sobald der Kamm oder die Hände, auf die das Stylingmittel aufgebracht wurde, mit den ersten Haarpartien in Kontakt kommen, werden vergleichsweise große Mengen an Stylingmittel an das Haar abgegeben. In Haarpartien, die erst später mit dem Kamm oder den Händen erreicht werden, wird hingegen vergleichsweise wenig Stylingmittel eingearbeitet. Dies hat zur Folge, dass der Anwender entweder von Anfang an eine große Menge Stylingmittel aufbringen muss, so dass auch die zuletzt erreichten Haarpartien ausreichend Stylingmittel erhalten, oder gezwungen ist, das Stylingmittel in mehreren Schritten aufzubringen, wobei jeweils andere Haarpartien behandelt werden. Haarsprays lassen sich gleichmäßiger auf das Haar verteilen. Da der Verwender aber keine Möglichkeit hat, die Gesamtmenge an aufgebrachtem Stylingmittel visuell zu erfassen, besteht die Gefahr, dass mehr Stylingmittel auf das Haar aufgebracht wird, als eigentlich erforderlich wäre.

Die bekannten Formen temporärer Stylingmittel erfordern zudem in der Regel eine große Menge an Hilfsstoffen, die nicht der eigentlichen Gestaltung der Frisur, sondern der Formulierung des jeweiligen Mittels dienen. So enthalten die Stylingmittel oftmals große Mengen organischer Lösungsmittel. Die Konfektionierung als Haarspray erfordert zudem weitere organische Verbindungen, die als Treibmittel Verwendung finden. Dies hat zum einen die Folge, dass die Umwelt mit flüchtigen organischen Verbindungen (VOC) belastet wird, zum anderen erhöht sich so das Produktvolumen und damit das Volumen der benötigten Verpackungen erheblich.

Aufgabe der vorliegenden Erfindung war es daher, ein Haarbehandlungsmittel zur temporären Formgebung zur Verfügung zur stellen, das einerseits Glanz und/oder ein hervorragendes und haltbares Stylingergebnis ergibt, und andererseits in möglichst kompakter Form vorliegt und genau und einfach dosiert werden kann.

Letzteres kann auf einfache Weise durch ein Stylingmittel erreicht werden, das in Pulverform vorliegt.

Pulverförmige Kosmetika sind bekannt und werden etwa im Bereich der Hautbehandlung bereits seit langem eingesetzt. Typische Beispiele sind etwa Make-up Puder oder Lidschatten. Um die pulverförmige Konsistenz zu erzielen, ist der Einsatz eines pulverförmigen Trägermaterials erforderlich. Als geeignetes Trägermaterial kann etwa Siliciumdioxid verwendet werden. Besonders interessant ist hydrophobiertes Siliciumdioxid. Dieses kann beispielsweise ausgehend von pyrogenem Siliciumdioxid, das in verschiedenen Spezifikationen kommerziell erhältlich ist, erhalten werden. Auf der Oberfläche trägt unbehandeltes pyrogenes Siliciumdioxid Silanol- und Siloxangruppen. Dadurch hat es eine hohe Affinität zu Wasser, d.h. es ist hydrophil. Durch Umsetzung mit geeigneten organischen Siliciumverbindungen lassen sich Alkylsilylgruppen auf der Oberfläche des pyrogenen Siliciumdioxids chemisch binden. Es entstehen modifizierte Siliciumdioxidpulver, die nicht mehr von Wasser benetzt werden, d.h. die hydrophobe Eigenschaften aufweisen.

In Seifen, Öle, Fette, Wachse (SÖFW), 3 (2004), S. 4-13 wird der Einsatz von hydrophobem Siliciumdioxid in der Kosmetik zur Herstellung von so genanntem trockenen Wasser für die Haut beschrieben. Dabei werden die hydrophoben Eigenschaften des modifizierten Siliciumdioxids ausgenutzt, die bewirken, dass das Siliciumdioxid beim intensiven Mischen mit Wasser nicht einfach in diesem dispergiert wird. Die Wassertropfen werden vielmehr von den hydrophoben Feststoffpartikeln umhüllt und am erneuten Zusammenfließen gehindert. Auf diese Weise lassen sich pulverförmige Feststoffe mit einem Wassergehalt von bis zu über 95 % erhalten. Unter mechanischer Beanspruchung, beispielsweise beim Verreiben auf der Haut, wird das eingeschlossene Wasser wieder freigesetzt. Dieses trockene Wasser wird als Grundlage zur Herstellung von lagerstabilem, festem Wasserstoffperoxid und von verstreichbaren Zubereitungen mit sehr geringem Ölgehalt beschrieben.

Dieses Konzept liegt auch der in EP 1 235 554 B1 beschriebenen Herstellung kosmetischer oder pharmazeutischer, verflüssigbarer Pulverzusammensetzungen zu Grunde. Die Pulverzusammensetzungen umfassen hydrophob beschichtete Metalloxidteilchen, in die Wasser und ein wasserlösliches Polymer eingeschlossen sind, wobei die Zusammensetzungen weniger als 1 % Öl enthalten. Durch Zugabe des wasserlöslichen Polymers soll erreicht werden, dass sich das Pulver bei der Anwendung auf der Haut angenehm und nicht körnig anfühlt, ohne dass zu diesem Zweck dem Produkt eine Ölkomponente zugegeben werden müsste. Das Polymer wird zu diesem Zweck der Wasserphase in einer Menge von 0,01 bis 5 Gew.-% zugegeben, wobei ein Gehalt an lediglich 0,1 bis 1 Gew.-% bevorzugt ist. Die verflüssigbaren Pulverzusammensetzungen werden vor allem zur Herstellung dekorativer Kosmetika eingesetzt. Daneben sind auch der Einsatz in Deodorants oder Sonnenschutzmitteln, oder die Anwendung auf dem Haar als Basis von Haarbehandlungsmitteln, die Perlglanzmittel oder Pflegekomponenten enthalten, beschrieben. Eine Verwendung im Bereich der Stylingmittel ist nicht genannt.

WO 03/037287 A1 offenbart die Verwendung eines Granulats auf Basis von pyrogenem Siliciumdioxid in kosmetischen Zusammensetzungen. Die speziellen Granulate können silanisiert, d.h. hydrophob werden und eignen sich zur Herstellung kosmetischer Zusammensetzungen jeglicher Konsistenz, beispielsweise Flüssigkeiten, Schäume, Sprays oder Pulver. Als mögliche kosmetische Zusammensetzungen werden eine Vielzahl denkbarer Kosmetika, unter anderem Haarstylingmittel, genannt. Dabei werden jedoch nur die üblichen Applikationsformen Lotion, Haarspray, Haarlack, Haargel und Haarwachs genannt. Ein Hinweis darauf, dass auf Basis des beschriebenen Siliciumdioxids pulverförmige Stylingmittel hergestellt werden könnten, findet sich nicht.

US 2003/0202993 offenbart pulverförmige Zubereitungen, die ein mit einem fluorhaltigen Mittel hydrophobiertes Metalloxidpulver enthalten und eine Ölkomponente, die sich zur Herstellung kosmetischer Zubereitungen wie Hair tonics eignen.

WO 2007/051511 offenbart pulverförmige Haarstylingmittel, die pulverförmiges hydrophobiertes Siliziumdioxid enthalten und mindestens ein filmbildendes und/oder festigendes Polymer sowie gegebenenfalls Öle.

Ein erster Gegenstand der vorliegenden Erfindung sind daher pulverförmige Zusammensetzungen zur temporären Verformung keratinischer Fasern, enthaltend
- 0 bis 20 Gew.-% Wasser,
- als Ölphase 40 Gew.-% bis 95 Gew.-% mindestens eines Öls,
- mindestens ein partikelförmiges, hydrophobiertes Metalloxid und
- mindestens ein filmbildendes und/oder festigendes Polymer.

Unter keratinischen Fasern sind dabei erfindungsgemäß Pelze, Wolle, Federn und insbesondere menschliche Haare zu verstehen.

Die pulverförmigen Zusammensetzungen können sehr einfach dosiert werden. Sie lassen sich zudem sehr gleichmäßig im Haar verteilen, da die Ölphase und gegebenenfalls darin befindliche Wirkstoffe erst unter mechanischer Beanspruchung freigesetzt werden. Das Pulver kann also zunächst vorsichtig im Haar verteilt und erst anschließend stärker mechanisch belastet werden, beispielsweise durch gezieltes Einmassieren des Pulvers in das Haar. Dadurch wird die Ölphase erst direkt auf der gewünschten Haarpartie freigesetzt. So lässt sich sehr gezielt eine hervorragende Dosierung und Wirkung erreichen.

Die Ölphase liegt in den erfindungsgemäßen pulverförmigen Zusammensetzungen in Form von Tröpfchen vor. Diese Tröpfchen sind an deren Oberfläche von den hydrophobierten Metalloxiden umgeben, so dass sich um die Tröpfchen herum eine Hülle, bestehend aus Partikeln des pulverförmigen hydrophobierten Metalloxids anordnet, die eine Koaleszenz der Tröpfchen verhindert. Die erfindungsgemäße pulverförmige Zusammensetzung umfasst demnach freie Partikel des pulverförmigen, hydrophobierten Metalloxids und an der Oberfläche der Tröpfchen der Ölphase adsorbierte Partikel des pulverförmigen, hydrophobierten Metalloxids.

Der Anteil von Wasser in der erfindungsgemäßen pulverförmigen Zusammensetzung beträgt erfindungsgemäß bevorzugt 0 bis 15 Gew.-%, besonders bevorzugt 0 bis 10 Gew.-%, ganz besonders bevorzugt 0 bis 5,5 Gew.-% - jeweils bezogen auf das Gewicht der gesamten Zusammensetzung.

Vorzugsweise wird eine pulverförmige Zusammensetzung verwendet, die 50 bis 90 Gew.-%, besonders bevorzugt 60 bis 90 Gew.-% - jeweils bezogen auf das Gewicht der gesamten Zusammensetzung - eines Öls als Ölphase enthält.

Unter einer Ölphase versteht sich erfindungsgemäß eine bei 20°C flüssige Phase, die sich bei 20°C zu weniger als 1 g in 100 g Wasser löst.

Die Ölphase besitzt bevorzugt eine Viskosität bis zu 250 mPas, (Brookfield, RVDV II+, 20°C, 20 Umdrehungen pro Minute, Spindel Nr. 1).

In einer bevorzugten Ausführungsform wird das Öl der Ölphase ausgewählt aus mindestens einem Öl der Gruppe, die gebildet wird aus
pflanzlichen Ölen,
flüssigen Paraffinölen,
Isoparaffinöten,
flüssigen synthetischen Kohlenwasserstoffen,
flüssigen Di-n-alkylethern,
Esterölen,
flüssigen Silikonen,
Dicarbonsäureestern,
symmetrischen, unsymmetrischen oder cyclischen Estern der Kohlensäure,
Mono,- Di- und Trifettsäureester von gesättigten und/oder ungesättigten linearen und/oder verzweigten C₆- bis C₂₂-Fettsäuren mit Glycerin.

Geeignet sind auch Triglyceridöle wie die flüssigen Anteile des Rindertalgs sowie synthetische Triglyceridöle.

Bevorzugte pflanzliche Öle werden ausgewählt aus mindestens einem Vertreter der Gruppe, die gebildet wird aus Amaranthöl, Sonnenblumenöl, Olivenöl, Sojaöl, Rapsöl, Mandelöl, Jojobaöl, Orangenöl, Aprikosenkernöl, Macadamianussöl, Weizenkeimöl, Pfirsichkernöl und die flüssigen Anteile des Kokosöls.

Bevorzugte Di-n-alkylether werden ausgewählt aus Di-n-alkylethern mit insgesamt zwischen 12 bis 36 C-Atomen, insbesondere 12 bis 24 C-Atomen, wie beispielsweise Di-n-octylether, Di-n-decylether, Di-n-nonylether, Di-n-undecylether, Di-n-dodecylether, n-Hexyl-n-octylether, n-Octyl-n-decylether, n-Decyl-n-undecylether, n-Undecyl-n-dodecylether und n-Hexyl-n-Undecylether sowie Di-tert-butylether, Di-iso-pentylether, Di-3-ethyldecylether, tert.-Butyl-n-octylether, iso-Pentyl-n-octylether und 2-Methyl-pentyl-n-octylether. Die als Handelsprodukte erhältlichen Verbindungen 1,3-Di-(2-ethyl-hexyl)-cyclohexan (Cetiol^{®} S) und Di-n-octylether (Cetiol^{®} OE) können bevorzugt sein.

Bevorzugte Esteröle werden ausgewählt aus Estern von C₆ - C₃₀ - Fettsäuren mit C₂ - C₃₀ - Fettalkoholen. Bevorzugt sind die Monoester der Fettsäuren mit Alkoholen mit 2 bis 24 C-Atomen. Beispiele für eingesetzte Fettsäurenanteile in den Estern sind Capronsäure, Caprylsäure, 2-Ethylhexansäure, Caprinsäure, Laurinsäure, Isotridecansäure, Myristinsäure, Palmitinsäure, Palmitoleinsäure, Stearinsäure, Isostearinsäure, Ölsäure, Elaidinsäure, Petroselinsäure, Linolsäure, Linolensäure, Elaeostearinsäure, Arachinsäure, Gadoleinsäure, Behensäure und Erucasäure sowie deren technische Mischungen, die z.B. bei der Druckspaltung von natürlichen Fetten und Ölen, bei der Oxidation von Aldehyden aus der Roelen'schen Oxosynthese oder der Dimerisierung von ungesättigten Fettsäuren anfallen. Beispiele für die Fettalkoholanteile in den Esterölen sind Isopropylalkohol, Capronalkohol, Caprylalkohol, 2-Ethylhexylalkohol, Caprinalkohol, Laurylalkohol, Isotridecylalkohol, Myristylalkohol, Cetylalkohol, Palmoleylalkohol, Stearylalkohol, Isostearylalkohol, Oleylalkohol, Elaidylalkohol, Petroselinylalkohol, Linolylalkohol, Linolenylalkohol, Elaeostearylalkohol, Arachylalkohol, Gadoleylalkohol, Behenylalkohol, Erucylalkohol und Brassidylalkohol sowie deren technische Mischungen, die z.B. bei der Hochdruckhydrierung von technischen Methylestern auf Basis von Fetten und Ölen oder Aldehyden aus der Roelen'schen Oxosynthese sowie als Monomerfraktion bei der Dimerisierung von ungesättigten Fettalkoholen anfallen. Erfindungsgemäß besonders bevorzugt sind Isopropylmyristat (Rilanit^{®} IPM), Isononansäure-C16-18-alkylester (Cetiol^{®} SN), 2-Ethylhexylpalmitat (Cegesoft^{®} 24), Stearinsäure-2-ethylhexylester (Cetiol^{®} 868), Cetyloleat, Glycerintricaprylat, Kokosfettalkohol-caprinat/-caprylat (Cetiol^{®} LC), n-Butylstearat, Oleylerucat (Cetiol^{®} J 600), Isopropylpalmitat (Rilanit^{®} IPP), Oleyl Oleate (Cetiol^{®}), Laurinsäurehexylester (Cetiol^{®} A), Di-n-butyladipat (Cetiol^{®} B), Myristylmyristat (Cetiol^{®} MM), Cetearyl Isononanoate (Cetiol^{®} SN), Ölsäuredecylester (Cetiol^{®} V).

Bevorzugte Dicarbonsäureester werden ausgewählt aus mindestens einer Verbindung der Gruppe, die gebildet wird, aus Di-n-butyladipat, Di-(2-ethylhexyl)-adipat, Di-(2-ethylhexyl)-succinat und Di-isotridecylacelaat sowie Diolestern wie Ethylenglykol-dioleat, Ethylenglykol-diisotridecanoat, Propylenglykol-di(2-ethylhexanoat), Propylenglykol-di-isostearat, Propylenglykol-di-pelargonat, Butandiol-di-isostearat, Neopentylglykoldicaprylat.

Insbesondere bevorzugt werden die flüssigen Silikone ausgewählt, aus mindestens einem Vertreter aus der Liste, die gebildet wird aus:
(i) Polyalkylsiloxanen, Polyarylsiloxanen, Polyalkylarylsiloxanen, die flüchtig oder nicht flüchtig, geradkettig, verzweigt oder cyclisch, vernetzt oder nicht vernetzt sind;
(ii) Polysiloxanen, die in ihrer allgemeinen Struktur eine oder mehrere organofunktionelle Gruppen enthalten, die ausgewählt sind unter:
   a) substituierten oder unsubstituierten aminierten Gruppen;
   b) (per)fluorierten Gruppen;
   c) Thiolgruppen;
   d) Carboxylatgruppen;
   e) hydroxylierten Gruppen;
   f) alkoxylierten Gruppen;
   g) Acyloxyalkylgruppen;
   h) amphoteren Gruppen;
   i) Bisulfitgruppen;
   j) Hydroxyacylaminogruppen;
   k) Carboxygruppen;
   l) Sulfonsäuregruppen; und
   m) Sulfat- oder Thiosulfatgruppen;
(iii) linearen Polysiloxan(A)- Polyoxyalkylen(B)- Blockcopolymeren vom Typ (A-B)ₙ mit n > 3;
(iv) gepfropften Silikonpolymeren mit nicht silikonhaltigem, organischen Grundgerüst, die aus einer organischen Hauptkette bestehen, welche aus organischen Monomeren gebildet wird, die kein Silikon enthalten, auf die in der Kette sowie gegebenenfalls an mindestens einem Kettenende mindestens ein Polysiloxanmakromer gepfropft wurde;
(v) oder deren Gemischen:

Besonders bevorzugte erfindungsgemäße Zubereitungen sind dadurch gekennzeichnet, dass sie als Öl mindestens ein Silikon der Formel (Si-1)

(CH₃)₃Si-[O-Si(CH₃)₂]ₓ-O-Si(CH₃)₃ (Si-1),

enthalten, in der x für eine Zahl von 0 bis 100, vorzugsweise von 0 bis 50, weiter bevorzugt von 0 bis 20 und insbesondere 0 bis 10, steht.

Die erfindungsgemäß bevorzugten pulverförmigen Zusammensetzungen enthalten als Öl mindestens ein Silikon der vorstehenden Formel (Si-1). Diese Silikone werden nach der INCI-Nomenklatur als Dimethicone bezeichnet. Es werden im Rahmen der vorliegenden Erfindung als Silikon der Formel (Si-1) vorzugsweise die Verbindungen:

(CH₃)₃Si-O-Si(CH₃)₃

(CH₃)₃Si-O-(CH₃)₂Si-O-Si(CH₃)₃

(CH₃)₃Si-[O-(CH₃)₂Si]₂-O-Si(CH₃)₃

(CH₃)₃Si-[O-(CH₃)₂Si]₃-O-Si(CH₃)₃

(CH₃)₃Si-[O-(CH₃)₂Si]₄-O-Si(CH₃)₃

(CH₃)₃Si-[O-(CH₃)₂Si]₅-O-Si(CH₃)₃

(CH₃)₃Si-[O-(CH₃)₂Si]₆-O-Si(CH₃)₃

(CH₃)₃Si-[O-(CH₃)₂Si]₇-O-Si(CH₃)₃

(CH₃)₃Si-[O-(CH₃)₂Si]₈-O-Si(CH₃)₃

(CH₃)₃Si-[O-(CH₃)₂Si]₉-O-Si(CH₃)₃

(CH₃)₃Si-[O-(CH₃)₂Si]₁₀-O-Si(CH₃)₃

(CH₃)₃Si-[O-(CH₃)₂Si]₁₁-O-Si(CH₃)₃

(CH₃)₃Si-[O-(CH₃)₂Si]₁₂-O-Si(CH₃)₃

(CH₃)₃Si-[O-(CH₃)₂Si]₁₃-O-Si(CH₃)₃

(CH₃)₃Si-[O-(CH₃)₂Si]₁₄-O-Si(CH₃)₃

(CH₃)₃Si-[O-(CH₃)₂Si]₁₅-O-Si(CH₃)₃

(CH₃)₃Si-[O-(CH₃)₂Si]₁₆-O-Si(CH₃)₃

(CH₃)₃Si-[O-(CH₃)₂Si]₁₇-O-Si(CH₃)₃

(CH₃)₃Si-[O-(CH₃)₂Si]₁₈-O-Si(CH₃)₃

(CH₃)₃Si-[O-(CH₃)₂Si]₁₉-O-Si(CH₃)₃

(CH₃)₃Si-[O-(CH₃)₂Si]₂₀-O-Si(CH₃)₃

eingesetzt, wobei (CH₃)₃Si-O-Si(CH₃)₃, (CH₃)₃Si-O-(CH₃)zSi-O-Si(CH₃)₃ und/oder (CH₃)₃Si-[O-(CH₃)₂Si]₂-O-Si(CH₃)₃ besonders bevorzugt sind.

Selbstverständlich können auch Mischungen der o.g. Silikone als Öl in den bevorzugten erfindungsgemäßen Mitteln enthalten sein.

Bevorzugte erfindungsgemäß einsetzbare Silikone weisen bei 20°C Viskositäten von 0,2 bis 2 mm²s⁻¹auf, wobei Silikone mit Viskositäten von 0,5 bis 1 mm²s⁻¹ besonders bevorzugt sind.

Besonders bevorzugte erfindungsgemäße pulverförmige Zusammensetzungen enthalten als Öl ein oder mehrere aminofunktionelle Silikone. Solche Silikone können z.B. durch die Formel (Si-2)

M(RₐQ_{b}SiO_{(4-a-b-)/2})ₓ(R_{c}SiO_{(-c)/2})_{y}M (Si-2)

Beschrieben werden, wobei in der obigen Formel
- R: ein Kohlenwasserstoff oder ein Kohlenwasserstoffrest mit 1 bis etwa 6 Kohlenstoffatomen ist,
- Q: ein polarer Rest der allgemeinen Formel -R¹HZ ist,
worin
- R¹: eine zweiwertige, verbindende Gruppe ist, die an Wasserstoff und den Rest Z gebunden ist, zusammengesetzt aus Kohlenstoff- und Wasserstoffatomen, Kohlenstoff-, Wasserstoff- und Sauerstoffatomen oder Kohlenstoff-, Wasserstoff- und Stickstoffatomen, und
- Z: ein organischer, aminofunktioneller Rest ist, der mindestens eine aminofunktionelle Gruppe enthält;
- a: Werte im Bereich von etwa 0 bis etwa 2 annimmt,
- b: Werte im Bereich von etwa 1 bis etwa 3 annimmt,
- a + b: kleiner als oder gleich 3 ist, und
- c: eine Zahl im Bereich von etwa 1 bis etwa 3 ist, und
- x: eine Zahl im Bereich von 1 bis etwa 2.000, vorzugsweise von etwa 3 bis etwa 50 und am bevorzugtesten von etwa 3 bis etwa 25 ist, und
- y: eine Zahl im Bereich von etwa 20 bis etwa 10.000, vorzugsweise von etwa 125 bis etwa 10.000 und am bevorzugtesten von etwa 150 bis etwa 1.000 ist, und
- M: eine geeignete Silikon-Endgruppe ist, wie sie im Stande der Technik bekannt ist, vorzugsweise Trimethylsiloxy.

Nicht einschränkende Beispiele der in Formel (Si-2) durch R repräsentierten Reste schließen Alkylreste, wie Methyl, Ethyl, Propyl, Isopropyl, Isopropyl, Butyl, Isobutyl, Amyl, Isoamyl, Hexyl, Isohexyl und ähnliche; Alkenylreste, wie Vinyl, Halogenvinyl, Alkylvinyl, Allyl, Halogenallyl, Alkylallyl; Cycloalkylreste, wie Cyclobutyl, Cyclopentyl, Cyclohexyl und ähnliche; Phenylreste, Benzylreste, Halogenkohlenwasserstoffreste, wie 3- Chlorpropyl, 4-Brombutyl, 3,3,3-Trifluorpropyl, Chlorcyclohexyl, Bromphenyl, Chlorphenyl und ähnliche sowie schwefelhaltige Reste, wie Mercaptoethyl, Mercaptopropyl, Mercaptohexyl, Mercaptophenyl und ähnliche ein; vorzugsweise ist R ein Alkylrest, der 1 bis etwa 6 Kohlenstoffatomen enthält, und am bevorzugtesten ist R Methyl. Beispiele von R¹ schließen Methylen, Ethylen, Propylen, Hexamethylen, Decamethylen, - CH₂CH(CH₃)CH₂-, Phenylen, Naphthylen, -CH₂CH₂SCH₂CH ₂-, -CH₂CH₂OCH₂-, -OCH₂CH₂-, - OCH₂ CH₂CH₂-, -CH₂CH(CH₃)C(O)OCH₂-, -(CH₂)₃ CC(O)OCH₂CH₂-, -C₆H ₄C₆H₄-, -C₆H ₄CH₂CₑH₄-; und -(CH ₂)₃C(O)SCH₂CH₂- ein.

Z ist gemäß Formel (Si-2) ein organischer, aminofunktioneller Rest, enthaltend mindestens eine funktionelle Aminogruppe. Eine mögliche Formel für besagtes Z ist NH(CH₂)_{z}NH₂, worin z eine ganze Zahl von größer gleich 1 ist. Eine andere mögliche Formel für besagtes Z ist -NH(CH₂)₂(CH ₂)_{zz}NH, worin sowohl z als auch zz unabhängig voneinander eine ganze Zahl von größer gleich 1 sind, wobei diese Struktur Diamino-Ringstrukturen umfasst, wie Piperazinyl. Besagtes Z ist am bevorzugtesten ein -NHCH₂CH ₂NH₂-Rest. Eine andere mögliche Formel für besagtes Z ist - N(CH₂)_{z}(CH₂)_{zz}NX₂ oder -NX₂, worin jedes X von X₂ unabhängig ausgewählt ist aus der Gruppe bestehend aus Wasserstoff und Alkylgruppen mit 1 bis 12 Kohlenstoffatomen, und zz 0 ist.

Q gemäß Formel (Si-2) ist am bevorzugtesten ein polarer aminofunktioneller Rest der Formel - CH₂CH₂CH₂NHCH₂CH₂NH₂.

In der Formel (Si-2) nimmt a Werte im Bereich von 0 bis 2 an, b nimmt Werte im Bereich von 2 bis 3 an, a + b ist kleiner als oder gleich 3, und c ist eine Zahl im Bereich von 1 bis 3. Das molare Verhältnis der RₐQ_{b} SiO(_{4-a-b)/2}-Einheiten zu den R_{c}SiO (_{4-c)/2}-Einheiten in Formel (Si-2) liegt im Bereich von etwa 1 : 2 bis 1 : 65, vorzugsweise von etwa 1 : 5 bis etwa 1 : 65 und am bevorzugtesten von etwa 1 : 15 bis etwa 1 : 20. Werden ein oder mehrere Silikone der obigen Formel (Si-2) eingesetzt, dann können die verschiedenen variablen Substituenten in der obigen Formel bei den verschiedenen Silikonkomponenten, die in der Silikonmischung vorhanden sind, verschieden sein.

Bevorzugte erfindungsgemäße pulverförmige Zubereitungen enthalten ein aminofunktionelles Silikon der Formel (Si-3)

R'ₐG₃₋ₐ-Si(OSiG ₂)ₙ-(OSiG _{b}R'_{2-b})ₘ-O-SiG₃₋ₐ-R'ₐ (Si-3),

worin bedeutet:
- G: ist -H, eine Phenylgruppe, -OH, -O-CH₃, -CH₃, -O-CH₂CH₃, -CH₂CH₃, -O-CH₂CH₂CH₃, -CH₂CH₂CH₃, -O-CH(CH₃)₂, -CH(CH₃)₂, -O-CH₂CH₂CH₂CH₃, -CH₂CH₂CH₂CH₃, -O-CH₂CH(CH₃)₂, -CH₂CH(CH₃)₂, -O-CH(CH₃)CH₂CH₃, -CH(CH₃)CH₂CH₃, -O-C(CH₃)₃, -C(CH₃)₃ ;
- a: steht für eine Zahl zwischen 0 und 3, insbesondere 0;
- b: steht für eine Zahl zwischen 0 und 1, insbesondere 1,
- m und n: sind Zahlen, deren Summe (m + n) zwischen 1 und 2000, vorzugsweise zwischen 50 und 150 beträgt, wobei n vorzugsweise Werte von 0 bis 1999 und insbesondere von 49 bis 149 und m vorzugsweise Werte von 1 bis 2000, insbesondere von 1 bis 10 annimmt,
- R': ist ein monovalenter Rest ausgewählt aus

-Q-N(R")-CH₂-CH₂-N(R")₂

-Q-N(R")₂

-Q-N⁺(R")₃A⁻

-Q-N⁺H(R")₂ A⁻

-Q-N⁺H₂(R")A⁻

-Q-N(R")-CH₂-CH₂-N⁺R"H₂A⁻ ,

wobei jedes Q für eine chemische Bindung, -CH₂-, -CH₂-CH₂-, -CH₂CH₂CH₂-, -C(CH₃)₂-, -CH₂CH₂CH₂CH₂-, -CH₂C(CH₃)₂-, -CH(CH₃)CH₂CH₂- steht, R" für gleiche oder verschiedene Reste aus der Gruppe -H, -Phenyl, -Benzyl, -CH₂-CH(CH₃)Ph, der C₁₋₂₀-Alkylreste, vorzugsweise -CH₃, -CH₂CH₃, -CH₂CH₂CH₃, -CH(CH₃)₂, -CH₂CH₂CH₂H₃, -CH₂CH(CH₃)₂, -CH(CH₃)CH₂CH₃, -C(CH₃)₃, steht und A ein Anion repräsentiert, welches vorzugsweise ausgewählt ist aus Chlorid, Bromid, Iodid oder Methosulfat.

Erfindungsgemäß geeignet sind kationische Silikonöle wie beispielsweise das Silikon der im Handel erhältlichen Dow Corning 929 Emulsion (enthaltend ein hydroxylamino-modifiziertes Silikon, das als Amodimethicone bezeichnet wird), DC 2-2078 (Hersteller Dow Corning, INCI-Bezeichnung: Aminopropyl Phenyl Trimethicone), DC 5-7113 (Hersteller Dow Corning, INCl-Bezeichnung: Silicone Quaternium 16), SM-2059 (Hersteller: General Electric), SLM-55067 (Hersteller: Wacker) sowie Abil^{®}-Quat 3270 und 3272 (Hersteller: Th. Goldschmidt; diquaternäre Polydimethylsiloxane, Quaternium-80).

Besonders bevorzugte erfindungsgemäße Ölphasen sind dadurch gekennzeichnet, dass sie als Öl mindestens es ein aminofunktionelles Silikon der Formel (Si3-a) 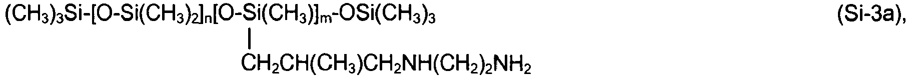 enthalten, worin m und n Zahlen sind, deren Summe (m + n) zwischen 1 und 2000, vorzugsweise zwischen 50 und 150 beträgt, wobei n vorzugsweise Werte von 0 bis 1999 und insbesondere von 49 bis 149 und m vorzugsweise Werte von 1 bis 2000, insbesondere von 1 bis 10 annimmt.

Diese Silikone werden nach der INCI-Deklaration als Trimethylsilylamodimethicone bezeichnet und sind beispielsweise unter der Bezeichnung Q2-7224 (Hersteller: Dow Corning; ein stabilisiertes Trimethylsilylamodimethicon) erhältlich.

Besonders bevorzugt sind auch erfindungsgemäße Ölphasen, die als Öl mindestens ein aminofunktionelles Silikon der Formel (Si-3b) 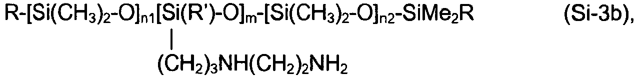 enthalten, worin
- R: für -OH, eine (gegebenenfalls ethoxylierte und/oder propoxylierte) (C₁ bis C₂₀)-Alkoxygruppe oder eine -CH₃-Gruppe steht,
- R': für -OH, eine (C₁ bis C₂₀)-Alkoxygruppe oder eine -CH₃-Gruppe und
- m, n1 und n2: Zahlen sind, deren Summe (m + n1 + n2) zwischen 1 und 2000, vorzugsweise zwischen 50 und 150 beträgt, wobei die Summe (n1 + n2) vorzugsweise Werte von 0 bis 1999 und insbesondere von 49 bis 149 und m vorzugsweise Werte von 1 bis 2000, insbesondere von 1 bis 10 annimmt.

Diese Silikone werden nach der INCI-Deklaration als Amodimethicone, bzw. als funktionalisierte Amodimethicone, wie beispielsweise Bis(C13-15 Alkoxy) PG Amodimethicone (beispielsweise als Handelsprodukt: DC 8500 der Firma Dow Corning erhältlich), Trideceth-9 PG-Amodimethicone (beispielsweise als Handelsprodukt Silcare Silicone SEA der Firma Clariant erhältlich) bezeichnet.

Unabhängig davon, welche aminofunktionellen Silikone eingesetzt werden, sind erfindungsgemäße Zubereitungen bevorzugt, die ein aminofunktionelles Silikon enthalten dessen Aminzahl oberhalb von 0,25 meq/g, vorzugsweise oberhalb von 0,3 meq/g und insbesondere oberhalb von 0,4 meq/g liegt. Die Aminzahl steht dabei für die Milli-Äquivalente Amin pro Gramm des aminofunktionellen Silikons. Sie kann durch Titration ermittelt und auch in der Einheit mg KOH/g angegeben werden.

Erfindungsgemäß bevorzugte Zubereitungen sind dadurch gekennzeichnet, dass sie in der Ölphase bezogen auf ihr Gewicht, 0,01 bis 10 Gew.%, vorzugsweise 0,1 bis 8 Gew.%, besonders bevorzugt 0,25 bis 7,5 Gew.% und insbesondere 0,5 bis 5 Gew.% aminofunktionelle(s) Silikon(e) enthalten.

Auch die nach INCI als Cyclomethicone bezeichneten cyclischen Dimethicone sind erfindungsgemäß mit Vorzug einsetzbar. Hier sind erfindungsgemäße Ölphasen bevorzugt, die als Öl mindestens ein Silikon der Formel (Si-4) 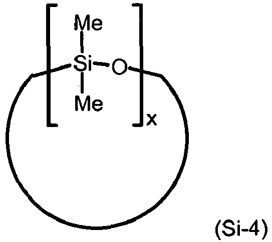 enthalten, in der x für eine Zahl von 0 bis 200, vorzugsweise von 0 bis 10, weiter bevorzugt von 0 bis 7 und insbesondere 0, 1, 2, 3, 4, 5 oder 6, steht.

Die vorstehend beschriebenen Silikone weisen ein Rückgrat auf, welches aus -Si-O-Si-Einheiten aufgebaut ist. Selbstverständlich können diese Si-O-Si-Einheiten auch durch Kohlenstoffketten unterbrochen sein. Entsprechende Moleküle sind durch Kettenverlängerungsreaktionen zugänglich.

Zusammenfassend umfasst dieses Herstellungsverfahren die emulgierende Mischung von Komponenten, deren eine mindestens ein Polysiloxane enthält, deren andere mindestens ein Organosilikonmaterial enthält, das mit dem Polysiloxane in einer Kettenverlängerungsreaktion reagiert, wobei mindestens ein Metallion-enthaltender Katalysator für die Kettenverlängerungsreaktion, mindestens ein Tensid und Wasser zugegen sind.

Kettenverlängerungsreaktionen mit Polysiloxanen sind bekannt und können beispielsweise die Hydrosilylierungsreaktion umfassen, in der eine Si-H Gruppe mit einer aliphatisch ungesättigten Gruppe in Gegenwart eines Platin/Rhodium-Katalysators unter Bildung von Polysiloxanes mit einigen Si-(C)ₚ-Si Bindungen (p = 1-6) reagiert, wobei die Polysiloxane auch als Polysiloxane-Polysilalkylene-Copolymere bezeichnet werden.

Die Kettenverlängerungsreaktion kann auch die Reaktion einer Si-OH Gruppe (beispielsweise eines Hydroxy-terminierten Polysiloxans) mit einer Alkoxygruppe (beispielsweise Alkoxysilanen, Silikaten oder Alkoxysiloxanen) in Gegenwart eines metallhaltigen Katalysators unter Bildung von Polysiloxanen umfassen.

Die Polysiloxane, die in der Kettenverlängerungsreaktion eingesetzt werden, umfassen ein substantiell lineares Polymer der folgenden Struktur:

R-Si(R₂)-[-O-Si(R₂)-]ₙ-O-SiR₃

In dieser Struktur steht jedes R unabhängig voneinander für einen Kohlenwasserstoffrest mit bis zu 20 Kohlenstoffatomen, vorzugsweise mit 1 bis 6 C-Atomen, wie beispielsweise einer Alkylgruppe (beispielsweise Methyl, Ethyl, Propyl oder Butyl), eine Arylgruppe (beispielsweise Phenyl), oder die für die Kettenverlängerungsreaktion benötigte Gruppe ("reaktive Gruppe", beispielsweise Si-gebundene H-Atome, aliphatisch ungesättigte Gruppen wie Vinyl, Allyl oder Hexenyl, Hydroxy, Alkoxy wie Methoxy, Ethoxy oder Propoxy, Alkoxy-Alkoxy, Acetoxy, Amino usw.), mit der Maßgabe, dass durchschnittlich ein bis zwei reaktive Gruppen pro Polymer vorliegen, n ist eine positive Zahl > 1. Vorzugsweise ist eine Mehrzahl der reaktiven Gruppen, besonders bevorzugt > 90%, und insbesondere > 98% der reaktiven Gruppen, an den endständigen Si-Atomen im Siloxan gebunden. Vorzugsweise steht n für Zahlen, die Polysiloxane beschreiben, welche Viskositäten zwischen 1 und 1.000.000 mm²/s besitzen, besonders bevorzugt Viskositäten zwischen 1.000 und 100.000 mm²/s.

Die Polysiloxane können zu einem geringen Grad verzweigt sein (beispielsweise < 2 Mol-% der Siloxaneinheiten), bzw. sind die Polymere aber substantiell linear, besonders bevorzugt vollständig linear. Zudem können die Substituenten R ihrerseits substituiert sein, beispielsweise mit N-haltigen Gruppen (beispielsweise Aminogruppen), Epoxygruppen, S-haltige Gruppen, Sihaltige Gruppen, O-haltige Gruppen usw.. Vorzugsweise sind mindestens 80% der Reste R Alkylreste, besonders bevorzugt Methylgruppen.

Das Organosilikonmaterial, das mit dem Polysiloxan in der Kettenverlängerungsreaktion reagiert, kann entweder ein zweites Polysiloxan sein, oder ein Molekül, das als Kettenverlängerer agiert. Wenn das Organosilikonmaterial ein Polysiloxan ist, hat es die vorstehend erwähnte generelle Struktur. In diesen Fällen besitzt ein Polysiloxan in der Reaktion (mindestens) eine reaktive Gruppe, und ein zweites Polysiloxan besitzt (mindestens) eine zweite reaktive Gruppe, die mit der ersten reagiert.

Falls das Organosilikonmaterial ein Kettenverlängerungs-Agens umfasst, kann dies ein Material sein wie beispielsweise ein Silan, ein Siloxan (beispielsweise Disiloxane oder Trisiloxan) oder ein Silazan. So kann beispielsweise eine Zusammensetzung, die ein Polysiloxan gemäß der vorstehend beschriebenen generellen Struktur umfasst, welches mindestens eine Si-OH Gruppe aufweist, ketteverlängert werden, indem mit einem Alkoxysilan (beispielsweise einem Dialkoxysilan oder Trialkoxysilan) in Gegenwart von Zinn- oder Titan-haltigen Katalysatoren reagiert wird.

Die metallhaltigen Katalysatoren in der Kettenverlängerungsreaktion sind meist spezifisch für eine bestimmte Reaktion. Solche Katalysatoren sind im Stand der Technik bekannt und enthalten beispielsweise Metalle wie Platin, Rhodium, Zinn, Titan, Kupfer, Blei, etc.. In einer bevorzugten Kettenverlängerungsreaktion wird ein Polysiloxan mit mindestens einer aliphatisch ungesättigten Gruppe, vorzugsweise einer Endgruppe, mit einem Organosilikonmaterial in Gegenwart eines Hydrosilylierungskatalysators zur Reaktion gebracht, das ein Siloxan oder Polysiloxan mit mindestens einer (vorzugsweise endständigen) Si-H Gruppe ist. Das Polysiloxan besitzt mindestens eine aliphatisch ungesättigte Gruppe und genügt der allgemeinen oben angegeben Formel, in der R und n wie vorstehend definiert sind, wobei im Durchschnitt zwischen 1 und 2 Gruppen R eine aliphatisch ungesättigte Gruppe pro Polymer besitzen. Repräsentative aliphatisch ungesättigte Gruppen sind beispielsweise Vinyl, Allyl, Hexenyl und Cyclohexenyl oder eine Gruppe R²CH=CHR³, in der R² für eine divalente aliphatische an das Silicium gebundene Kette und R³ für ein Wasserstoffatom oder eine Alkylgruppe steht. Das Organosilikonmaterial mit mindestens einer Si-H Gruppe hat vorzugsweise die oben genannte Struktur, in der R und n wie vorstehend definiert sind und wobei im Durchschnitt zwischen 1 und 2 Gruppen R ein Wasserstoff bedeuten und n 0 oder eine positive ganze Zahl ist.

Dieses Material kann ein Polymer oder ein niedermolekulares Material wie ein Siloxan sein (beispielsweise ein Disiloxane oder ein Trisiloxan).

Das Polysiloxan mit mindestens einer aliphatisch ungesättigten Gruppe und das Organosilikonmaterial mit mindestens einer Si-H Gruppe reagieren in Gegenwart eines Hydrosilylierungskatalysators. Solche Katalysatoren sind aus dem Stand der Technik bekannt und umfassen beispielsweise Platin- und Rhodium-enthaltende Materialien. Die Katalysatoren können jede bekannte Form annehmen, beispielsweise auf Trägermaterialien (wie beispielsweise Silica Gel oder Aktivkohle) aufgebrachtes Platin oder Rhodium oder andere geeignete Compounds wie Platinchlorid, Salze von Platin- oder Chloroplatinsäuren. Ein wegen der guten Dispergierbarkeit in Organosilikonsystemen und der geringen Farbveränderung bevorzugter Katalysator ist Chloroplatinsäure entweder als kommerziell verfügbares Hexahydrat oder in wasserfreier Form.

Bei einer weiteren bevorzugten Kettenerweiterungsreaktion wird ein Polysiloxan mit mindestens einer Si-OH Gruppe, vorzugsweise einer Endgruppe, mit einem Organosilikonmaterial zu Reaktion gebracht, das mindestens eine Alkoxygruppe besitzt, vorzugsweise ein Siloxan mit mindestens einer Si-OR Gruppe oder ein Alkoxysilan mit mindestens zwei Alkoxygruppen. Hierbei wird als Katalysator wieder ein metallhaltiger Katalysator eingesetzt.

Für die Reaktion einer Si-OH Gruppe mit einer Si-OR Gruppe existieren viele literaturbekannte Katalysatoren, beispielsweise Organometallverbindungen wie Organozinnsalze, Titanate oder Titanchelate bzw. -komplexe. Beispiele umfassen Zinn-octoat, Dibutylzinn-dilaurat, Dibutylzinndiacetat, Dimethyltinn-dineodecanoat, Dibutylzinn-dimethoxid, Isobutylzinn-triceroat, Dimethylzinn-dibutyrat, Dimethylzinn-dineodecanoat, Triethylzinn-tartrat, Zinnoleat, Zinnnaphthenat, Zinnbutyrat, Zinnacetat, Zinnbenzoat, Zinnsebacat, Zinnsuccinat, Tetrabutyltitanat, Tetraisopropyltitante, Tetraphenyltitanat, Tetraoctadecyltitanat, Titannaphthanat, Ethyltriethanolamin-Titanat, Titani-diisopropyl-diethyl-acetoacetat, Titan-diisopropoxydiacetyl-acetonat und Titani-tetra-Alkoxide, bei denen das Alkoxid Butoxy oder Propoxy ist.

Erfindungsgemäß ebenfalls bevorzugte Mittel sind dadurch gekennzeichnet, dass sie in der Ölphase mindestens ein Silikon der Formel (Si-5)

R₃Si-[O-SiR₂]ₓ-(CH₂)ₙ-[O-SiR₂]_{y}-O-SiR₃ (Si-5),

enthalten, in der R für gleiche oder verschiedene Reste aus der Gruppe -H, -Phenyl, -Benzyl, -CH₂-CH(CH₃)Ph, der C₁₋₂₀-Alkylreste, vorzugsweise -CH₃, -CH₂CH₃, -CH₂CH₂CH₃, -CH(CH₃)₂, -CH₂CH₂CH₂H₃, -CH₂CH(CH₃)₂, -CH(CH₃)CH₂CH₃, -C(CH₃)₃, steht, x bzw. y für eine Zahl von 0 bis 200, vorzugsweise von 0 bis 10, weiter bevorzugt von 0 bis 7 und insbesondere 0, 1, 2, 3, 4, 5 oder 6, stehen, und n für eine Zahl von 0 bis 10, bevorzugt von 1 bis 8 und insbesondere für 2, 3, 4, 5, 6 steht.

Bevorzugt als Öl der Ölphase einsetzbare symmetrische, unsymmetrische oder cyclische Ester der Kohlensäure mit Fettalkoholen werden in der DE-OS 197 56 454 beschrieben und sind Glycerincarbonat, Propylencarbonat und/oder Dicaprylylcarbonat (Cetiol^{®} CC).

Bevorzugt als Öl in der Ölphase verwendbare Mono,- Di- und Trifettsäureester von gesättigten und/oder ungesättigten linearen und/oder verzweigten Fettsäuren mit Glycerin sind die Ester der Handelsprodukte Monomuls^{®} 90-018, Monomuls^{®} 90-L12 oder Cutina^{®} MD.

Es kann zur Glanz bzw. Haltverbesserung vorteilhaft sein, wenn in die Ölphase mindestens ein Fettstoff eingearbeitet wird.

Unter einem Fettstoff werden erfindungsgemäß solche Verbindungen verstanden, die bei 20°C zu weniger als 1 g in 100 g Wasser löslich sind und eine Viskosität von mehr als 23000 mPas (Brookfield, 20°C, 5 Umdrehungen pro Minute, Spindel TE mit Helipath) aufweisen.

Bevorzugte Fettstoffe sind ausgewählt aus mindestens einem Fettstoffe der Gruppe, die gebildet wird aus festen Silikonen, pastösen Silikonen.

Die pulverförmigen Zusammensetzungen enthalten als weiteren wichtigen Bestandteil mindestens ein partikelförmiges, hydrophobiertes Metalloxid.

Die erfindungsgemäß pulverförmige Zusammensetzung enthält die partikelförmigen, hydrophobierten Metalloxide bevorzugt in einer Menge von 0,5 Gew.-% bis 15 Gew.-% - bezogen auf das Gewicht der gesamten pulverförmigen Zusammensetzung.

Ferner hat es sich als vorteilhaft erwiesen, wenn die partikelförmigen, hydrophobierten Metalloxide einen Partikeldurchmesser von weniger als 5 µm, bevorzugt weniger als 1 µm, besonders bevorzugt zwischen 20 und 100 nm, aufweisen

Die Metalloxide werden bevorzugt ausgewählt aus Aluminaten, Silikaten, Aluminiumsilikaten, Titandioxid sowie Silikagel, insbesondere unter Aluminaten, Silikaten, Aluminiumsilikaten sowie Silkagel.

Besonders bevorzugte Aluminate werden ausgewählt unter aktivem Aluminiumoxid, alpha-Aluminiumoxid, beta-Aluminiumoxid, gamma-Aluminiumoxid sowie deren Gemischen.

Besonders bevorzugte Aluminiumsilikate (auch Alumosilikate genannt) werden ausgewählt unter Phyllosilikaten, Tectosilikaten.

Bevorzugt geeignete Phyllosilikate werden ausgewählt unter Kaolinen (hier insbesondere unter Kaolinit, Dickit, Hallosit sowie Nakrit), Serpentin, Talk, Pyrophyllit, Montmorillonit, Quarz, Bentonit, Glimmer (hier insbesondere unter Illit, Muscovit, Paragonit, Phlogopit, Biotit, Lepidolith, Margarit, Smektit (hier insbesondere unter Montmorrilionit, Saponit, Nontronit, Hectorit)).

Bevorzugt geeignete Tectosilikate werden ausgewählt unter Feldspatmineralien (insbesondere Albit, Orthoklas, Anorthit, Leucit, Sodalith, Hauyn, Labradorit, Lasurit, Nosean, Nephelin).

Besonders zur Ausführung der Erfindung geeignete partikelförmige, hydrophobierte Metalloxide sind solche, die an der Oberfläche mit perfluorierten Verbindungen, insbesondere mit Perfluoralkylresten, hydrophobiert wurden. Dabei sind wiederum solche partikelförmigen, hydrophobierten Metalloxide bevorzugt, die mit mindestens einem Rest hydrophobiert wurden, ausgewählt aus Perfluoralkylsilyl, Polyperfluoralkylenoxid, Perfluoralkylpolyalkylenoxid, Polysiloxan mit Perfluoralkylgruppen, Perfluoralkylphosphat, Polyfluoralkylphosphatether.

Dabei ist es wiederum besonders bevorzugt, wenn das partikelförmige, hydrophobierte Metalloxid ausgewählt wird, aus mindestens einem Vertreter der Gruppe die gebildet wird aus fluoriertem Phyllosilikat (insbesondere fluoriertem Talk, fluoriertem Glimmer), mit Perfluoralkylphosphat hydrophobiertem Phyllosilikat (insbesondere mit Perfluoralkylphosphat hydrophobiertem Talk, mit Perfluoralkylphosphat hydrophobiertem Glimmer), mit Perfluoralkylphosphat hydrophobiertem Titandioxid und mit Perfluoralkylphosphat hydrophobiertem Siliziumdioxid.

Ganz besonders bevorzugt sind dabei die Handelsprodukte PW Covafluor^{®} (Firma LCW), PFS Talc JA 46-R^{®} (Firma Daito), Talc JA R46PF^{®} (Firma LCW), Submica M^{®} (Firma LCW).

Zur Verbesserung der erfindungsgemäßen Effekte wird zusätzlich mindestens ein filmbildendes und/oder festigendes Polymer zugesetzt werden. Dabei ist es bevorzugt, wenn diese filmbildenden und/oder festigenden Polymere - insbesondere die nachfolgend aufgeführten, bevorzugten Vertreter - in der Ölphase dispergiert oder gelöst vorliegen.

Das filmbildende und/oder festigende Polymer ist im Rahmen dieser Ausführungsform in der pulverförmigen Zusammensetzung vorzugsweise in einer Menge von 1 bis 15 Gewichtsprozent, besonders bevorzugt von 5,5 bis 15 Gewichtsprozent, ganz besonders bevorzugt in einer Menge von 6 bis 10 Gewichtsprozent, bezogen auf die gesamte pulverförmige Zusammensetzung, enthalten. Selbstverständlich können auch mehrere filmbildende und/oder festigende Polymere enthalten sein. Dabei können diese filmbildenden und/oder festigenden Polymere sowohl permanent als auch temporär kationisch, anionisch, nichtionisch oder amphoter sein. Bei der Verwendung von mindestens zwei filmbildenden und/oder festigenden Polymeren können diese selbstverständlich unterschiedliche Ladungen aufweisen. Erfindungsgemäß bevorzugt kann es sein, wenn ein ionisches filmbildendes und/oder festigendes Polymer mit einem amphoteren und/oder nichtionischen filmbildenden und/oder festigenden Polymer gemeinsam verwendet wird. Auch die Verwendung mindestens zweier gegensätzlich geladener film bildender und/oder festigender Polymere ist bevorzugt. In letzterem Falle kann eine besondere Ausführungsform wiederum zusätzlich mindestens ein weiteres amphoteres und/oder nichtionisches filmbildendes und/oder festigendes Polymer enthalten.

Da Polymere häufig multifunktional sind, können deren Funktionen nicht immer klar und eindeutig voneinander abgegrenzt werden. Insbesondere gilt dies für filmbildende und festigende Polymere. Viele Polymere, die primär als filmbildend beschrieben werden, haben auch festigende Eigenschaften und umgekehrt. Es wird an dieser Stelle daher explizit darauf verwiesen, dass im Rahmen der vorliegenden Erfindung sowohl filmbildende als auch festigende Polymere wesentlich sind. Da beide Eigenschaften nicht völlig unabhängig voneinander sind, werden unter dem Begriff "festigende Polymere" auch immer "filmbildende Polymere" verstanden und umgekehrt.

Zu den bevorzugten Eigenschaften der filmbildenden Polymeren zählt die Filmbildung. Unter filmbildenden Polymeren sind solche Polymere zu verstehen, welche beim Trocknen einen kontinuierlichen Film auf der Haut, dem Haar oder den Nägeln hinterlassen. Derartige Filmbildner können in den unterschiedlichsten kosmetischen Produkten wie beispielsweise Gesichtsmasken, Make-up, Haarfestigem, Haarsprays, Haargelen, Haarwachsen, Haarkuren, Shampoos oder Nagellacken verwendet werden. Bevorzugt sind insbesondere solche Polymere, die eine ausreichende Löslichkeit in Wasser, Alkohol oder Wasser/Alkohol-Gemischen besitzen. So lassen sich entsprechende Lösungen herstellen, die sich auf einfache Art und Weise anwenden bzw. weiterverarbeiten lassen. Die filmbildenden Polymere können synthetischen oder natürlichen Ursprungs sein.

Unter filmbildenden Polymeren werden weiterhin solche Polymere verstanden, die bei Anwendung in 0,01 bis 20 Gew.-%-iger wässriger, alkoholischer oder wässrigalkoholischer Lösung in der Lage sind, auf dem Haar einen transparenten Polymefilm abzuscheiden. Die filmbildenden Polymere können dabei sowohl anionisch, amphoter, nicht-ionisch, permanent kationisch oder temporär kationisch geladen sein.

Geeignete und erfindungsgemäß bevorzugt eingesetzte synthetische, filmbildende, haarfestigende Polymere sind Homo- oder Copolymere, die aus mindestens einem der folgenden Monomere aufgebaut sind: Vinylpyrrolidon, Vinylcaprolactam, Vinylester wie z.B. Vinylacetat" Vinylalkohol, Acrylamid, Methacrylamid, C₁- bis C₇-Alkylacrylamid, C₁- bis C₇-Dialkylacrylamid, C₁- bis C₇-Alkylmethacrylamid, C₁- bis C₇-Dialkylmethacrylamid, C₁- bis C₇-Alkylacrylat, Acrylsäure, Propylenglykol, Ethylenglykol, wobei die C₁- bis C₇-Alkylgruppen dieser Monomere vorzugsweise C₁- bis C₃-Alkylgruppen sind.

Beispielhaft seien genannt Homopolymere des Vinylcaprolactams, des Vinylpyrrolidons oder des N-Vinylformamids. Weitere geeignete synthetische filmbildende, haarfestigende Polymere sind z.B. Copolymerisate aus Vinylpyrrolidon und Vinylacetat, Terpolymere aus Vinylpyrrolidon, Vinylacetat und Vinylpropionat, Polyacrylamide, die beispielsweise unter den Handelsbezeichnungen Akypomine^{®} P 191 von der Firma CHEM-Y, Emmerich, oder Sepigel^{®} 305 von der Firma Seppic vertrieben werden; Polyvinylalkohole, die beispielsweise unter den Handelsbezeichnungen Elvanol^{®} von Du Pont oder Vinol^{®} 523/540 von der Firma Air Products vertrieben werden sowie Polyethylenglykol/Polypropylenglykol-Copolymere, die beispielsweise, unter den Handelsbezeichnungen Ucon^{®} der Union Carbide vertrieben werden.

Geeignete natürliche filmbildende Polymere sind z.B. Cellulosederivate, z. B. Hydroxypropylcellulose mit einem Molekulargewicht von 30.000 bis 50.000 g/mol, welche beispielsweise unter der Handelsbezeichnung Nisso SI^{®} von der Firma Lehmann & Voss, Hamburg, vertrieben wird.

Festigende Polymere tragen zum Halt und/oder zum Aufbau des Haarvolumens und der Haarfülle der Gesamtfrisur bei. Diese sogenannten festigenden Polymere sind gleichzeitig auch filmbildende Polymere und daher generell typische Substanzen für formgebende Haarbehandlungsmittel wie Haarfestiger, Haarschäume, Haarwachse, Haarsprays. Die Filmbildung kann dabei durchaus punktuell sein und nur einige Fasern miteinander verbinden.

Substanzen, welche dem Haar weiterhin hydrophobe Eigenschaften verleihen, sind hierbei bevorzugt, weil sie die Tendenz des Haares, Feuchtigkeit, also Wasser, zu absorbieren, verringern. Dadurch wird das schlaffe Herunterhängen der Haarsträhnen vermindert und somit ein langanhaltender Frisurenaufbau und -erhalt gewährleistet. Als Testmethode hierfür wird häufig der sogenannte curl-retention - Test angewendet. Diese polymeren Substanzen können weiterhin erfolgreich in leave-on und rinse-off Haarkuren oder Shampoos eingearbeitet werden. Da Polymere häufig multifunktional sind, das heißt mehrere anwendungstechnisch erwünschte Wirkungen zeigen, finden sich zahlreiche Polymere in mehreren nach der jeweiligen Wirkungsweise eingeteilten Gruppen, so auch im CTFA Handbuch. Wegen der Bedeutung gerade der festigenden Polymere sollen diese daher explizit in Form ihrer INCI - Namen aufgelistet werden. In dieser Liste finden sich somit selbstverständlich gerade auch die genannten filmbildenden Polymere wieder.

Beispiele für gebräuchliche filmbildende, festigende Polymere sind Acrylamide/Ammonium Acrylate Copolymer, Acrylamides/DMAPA Acrylates/Methoxy PEG Methacrylate Copolymer, Acrylamidopropyltrimonium Chloride/Acrylamide Copolymer, Acrylamidopropyltrimonium Chloride/Acrylates Copolymer, Acrylates/Acetoacetoxyethyl Methacrylate Copolymer, Acrylates/Acrylamide Copolymer, Acrylates/Ammonium Methacrylate Copolymer, Acrylates/t-Butylacrylamide Copolymer, Acrylates Copolymer, Acrylates/C1-2 Succinates/Hydroxyacrylates Copolymer, Acrylates/Lauryl Acrylate/Stearyl Acrylate/Ethylamine Oxide Methacrylate Copolymer, Acrylates/Octylacrylamide Copolymer, Acrylates/Octylacrylamide/Diphenyl Amodimethicone Copolymer, Acrylates/Stearyl Acrylate/Ethylamine Oxide Methacrylate Copolymer, Acrylates/VA Copolymer, Acrylates/VP Copolymer, Adipic Acid/Diethylenetriamine Copolymer, Adipic Acid/Dimethylaminohydroxypropyl Diethylenetriamine Copolymer, Adipic Acid/Epoxypropyl Diethylenetriamine Copolymer, Adipic Acid/Isophthalic Acid/Neopentyl Glycol/Trimethylolpropane Copolymer, Allyl Stearate/VA Copolymer, Aminoethylacrylate Phosphate/Acrylates Copolymer, Aminoethylpropanediol-Acrylates/Acrylamide Copolymer, Aminoethylpropanediol-AMPD-Acrylates/Diacetoneacrylamide Copolymer, Ammonium VA/Acrylates Copolymer, AMPD-Acrylates/Diacetoneacrylamide Copolymer, AMP-Acrylates/Allyl Methacrylate Copolymer, AMP-Acrylates/C1-18 Alkyl Acrylates/C1-8 Alkyl Acrylamide Copolymer, AMP-Acrylates/Diacetoneacrylamide Copolymer, AMP-Acrylates/Dimethylaminoethylmethacrylate Copolymer, Bacillus/Rice Bran Extract/Soybean Extract Ferment Filtrate, Bis-Butyloxyamodimethicone/PEG-60 Copolymer, Butyl Acrylate/Ethylhexyl Methacrylate Copolymer, Butyl Acrylate/Hydroxypropyl Dimethicone Acrylate Copolymer, Butylated PVP, Butyl Ester of Ethylene/MA Copolymer, Butyl Ester of PVM/MA Copolymer, Calcium/Sodium PVM/MA Copolymer, Corn Starch/Acrylamide/ Sodium Acrylate Copolymer, Diethylene Glycolamine/Epichlorohydrin/Piperazine Copolymer, Dimethicone Crosspolymer, Diphenyl Amodimethicone, Ethyl Ester of PVM/MA Copolymer, Hydrolyzed Wheat Protein/PVP Crosspolymer, Isobutylene/Ethylmaleimide/ Hydroxyethylmaleimide Copolymer, Isobutylene/MA Copolymer, Isobutylmethacrylate/Bis-Hydroxypropyl Dimethicone Acrylate Copolymer, Isopropyl Ester of PVM/MA Copolymer, Lauryl Acrylate Crosspolymer, Lauryl Methacrylate/Glycol Dimethacrylate Crosspolymer, MEA-Sulfite, Methacrylic Acid/Sodium Acrylamidomethyl Propane Sulfonate Copolymer, Methacryloyl Ethyl Betaine/Acrylates Copolymer, Octylacrylamide/Acrylates/Butylaminoethyl Methacrylate Copolymer, PEG/PPG-25/25 Dimethicone/Acrylates Copolymer, PEG-8/SMDI Copolymer, Polyacrylamide, Polyacrylate-6, Polybeta-Alanine/Glutaric Acid Crosspolymer, Polybutylene Terephthalate, Polyester-1, Polyethylacrylate, Polyethylene Terephthalate, Polymethacryloyl Ethyl Betaine, Polypentaerythrityl Terephthalate, Polyperfluoroperhydrophenanthrene, Polyquaternium-1, Polyquaternium-2, Polyquaternium-4, Polyquatemium-5, Polyquaternium-6, Polyquaternium-7, Polyquaternium-8, Polyquaternium-9, Polyquatemium-10, Polyquaternium-11, Polyquaternium-12, Polyquaternium-13, Polyquaternium-14, Polyquaternium-15, Polyquaternium-16, Polyquaternium-17, Polyquaternium-18, Polyquaternium-19, Polyquaternium-20, Polyquaternium-22, Polyquaternium-24, Polyquaternium-27, Polyquaternium-28, Polyquaternium-29, Polyquaternium-30, Polyquaternium-31, Polyquaternium-32, Polyquaternium-33, Polyquaternium-34, Polyquaternium-35, Polyquaternium-36, Polyquaternium-37, Polyquaternium-39, Polyquaternium-45, Polyquaternium-46, Polyquaternium-47, Polyquaternium-48, Polyquaternium-49, Polyquaternium-50, Polyquaternium-55, Polyquaternium-56, Polysilicone-9, Polyurethane-1, Polyurethane-6, Polyurethane-10, Polyvinyl Acetate, Polyvinyl Butyral, Polyvinylcaprolactam, Polyvinylformamide, Polyvinyl Imidazolinium Acetate, Polyvinyl Methyl Ether, Potassium Butyl Ester of PVM/MA Copolymer, Potassium Ethyl Ester of PVM/MA Copolymer, PPG-70 Polyglyceryl-10 Ether, PPG-12/SMDI Copolymer, PPG-51/SMDI Copolymer, PPG-10 Sorbitol, PVM/MA Copolymer, PVP, PVPNA/Itaconic Acid Copolymer, PVP/VA/Vinyl Propionate Copolymer, Rhizobian Gum, Rosin Acrylate, Shellac, Sodium Butyl Ester of PVM/MA Copolymer, Sodium Ethyl Ester of PVM/MA Copolymer, Sodium Polyacrylate, Sterculia Urens Gum, Terephthalic Acid/Isophthalic Acid/Sodium Isophthalic Acid Sulfonate/Glycol Copolymer, Trimethylolpropane Triacrylate, Trimethylsiloxysilylcarbamoyl Pullulan, VA/Crotonates Copolymer, VA/Crotonates/Methacryloxybenzophenone-1 Copolymer, VA/Crotonates/Vinyl Neodecanoate Copolymer, VA/Crotonates/Vinyl Propionate Copolymer, VA/DBM Copolymer, VA/Vinyl Butyl Benzoate/Crotonates Copolymer, Vinylamine/Vinyl Alcohol Copolymer, Vinyl Caprolactam/VP/Dimethylaminoethyl Methacrylate Copolymer, VP/Acrylates/Lauryl Methacrylate Copolymer, VP/Dimethylaminoethylmethacrylate Copolymer, VP/DMAPA Acrylates Copolymer, VP/Hexadecene Copolymer, VPNA Copolymer, VP/Vinyl Caprolactam/DMAPA Acrylates Copolymer, Yeast Palmitate.

Vorzugsweise werden erfindungsgemäß pulverförmige Zusammensetzungen verwendet, die mindestens ein filmbildendes und/oder festigendes Polymer enthalten, das aus Vinylpyrrolidon-Vinylacetat-Copolymeren, Vinylacetat-Crotonsäure-Copolymeren, Vinylcaprolactam-Vinylpyrrolidon-Dimethylaminoethylmethacrylat-Copolymeren, Octylacrylamid-Acrylat-Butylaminoethyl-Methacrylat-Copolymeren und quaternierten Vinylpyrrolidon-Dimethylaminoethylmethacrylat-Copolymeren ausgewählt ist.

Besonders bevorzugt handelt es sich bei dem filmbildenden und/oder festigenden Polymer um die Vinylpyrrolidon-Vinylacetat-Copolymeren Luviskol^{®} VA 37 oder PVP/VA Copolymer 60/40 W NP, das Vinylacetat-Crotonsäure-Copolymere, das unter der Handelsbezeichnung Aristoflex^{®} A 60 vertrieben wird, das Vinylcaprolactam-Vinylpyrrolidon-Dimethylaminoethylmethacrylat-Copolymer mit der Handelsbezeichnung Advantage^{®} LC-E, das unter der Bezeichnung Amphomer^{®} erhältliche amphotere Octylacrylamid-Acrylat-Butylaminoethyl-Methacrylat-Copolymer oder das durch Umsetzung mit Diethylsulfat quatemierte Vinylpyrrolidon-Dimethylaminoethylmethacrylat-Copolymer, das unter der Handelsbezeichnung Gafquat^{®} 755N vertrieben wird.

Insbesondere bevorzugt werden pulverförmige Zusammensetzungen eingesetzt, die zusätzlich wenigstens ein Vinylpyrrolidon-Vinylacetat-Copolymer enthalten.

Des weiteren eignet sich erfindungsgemäß bevorzugt der Einsatz mindestens eines Verdickers für die Ölphase. Hierzu werden bevorzugt Polymere eingesetzt, welche nicht wasserlöslich aber kompatibel mit Lipiden sind. Solche Verdicker werden bevorzugt ausgewählt aus mindestens einem Vertreter der Gruppe, die gebildet wird aus Acrylates/C10-30 Alkyl Acrylate Crosspolymer, Adipic Acid/PPG-10 Copolymer, Allyl Methacrylates Crosspolymer, Alumina Magnesium Metasilicate, Aluminum Starch Octenylsuccinate, Beeswax, Behenyl Methacrylate/Perfluorooctylethyl Methacrylate Copolymer, Bispolyethylene Dimethicone, Butadiene/Acrylonitrile Copolymer, Butylene/Ethylene Copolymer, Butylene/Ethylene/Styrene Copolymer, Butylene Glycol Montanate, Butyrospermum Parkii (Shea Butter), C29-70 Acid, C23-43 Acid Pentaerythritol Tetraester, C20-24 Alkyl Dimethicone, C24-28 Alkyl Dimethicone, C1-5 Alkyl Galactomannan, C18-38 Alkyl Hydroxystearoyl Stearate, C20-24 Alkyl Methicone, C24-28 Alkyl Methicone, C30-45 Alkyl Methicone, Candelilla Wax Hydrocarbons, C10-30 Cholesterol/Lanosterol Esters, Cellobiose Octanonanoate, Ceresin, Cerotic Acid, Cetearyl Dimethicone/Vinyl Dimethicone Crosspolymer, Chlorinated Paraffin, Cholesterol, Cholesteryl Acetate, Cholesteryl Hydroxystearate, Cholesteryl Isostearate, Cholesteryl Macadamiate, Cholesteryl Stearate, C10-40 Hydroxyalkyl Acid Cholesterol Esters, C10-40 Isoalkyl Acid Cholesterol Esters, C10-40 Isoalkyl Acid Octyldodecanol Esters, C10-40 Isoalkyl Acid Phytosterol Esters, C10-40 Isoalkyl Acid Triglyceride, C30-38 Olefin/Isopropyl Maleate/MA Copolymer, Copal, Corn Starch Modified, C6-14 Perfluoroalkylethyl Acrylate/HEMA Copolymer, C6-14 Polyolefin, Decene/Butene Copolymer, Dihydrogenated Tallow Benzylmonium Hectorite, Dilinoleic Acid/Ethylenediamine Copolymer, Dilinoleic Acid/Sebacic Acid/Piperazine/Ethylenediamine Copolymer, Dimethicone Crosspolymer, Dimethicone/Phenyl Vinyl Dimethicone Crosspolymer, Dimethicone/Vinyl Dimethicone Crosspolymer, Dimethicone/Vinyltrimethylsiloxysilicate Crosspolymer, Diphenyl Dimethicone/Vinyl Diphenyl Dimethicone/Silsesquioxane Crosspolymer, Divinyldimethicone/Dimethicone Crosspolymer, Dodecanedioic Acid/Cetearyl Alcohol/Glycol Copolymer, Ethylcellulose, Ethylene/Acrylic Acid Copolymer, Ethylene/Acrylic Acid/VA Copolymer, Ethylenediamine/Dimer Tallate Copolymer Bis-Hydrogenated Tallow Amide, Ethylenediamine/Stearyl Dimer Dilinoleate Copolymer, Ethylenediamine/Stearyl Dimer Tallate Copolymer, Ethylene/Octene Copolymer, Ethylene/Propylene Copolymer, Ethylene/Propylene/Styrene Copolymer, Euphorbia Cerifera (Candelilla) Wax, Hydrogenated Butylene/Ethylene/Styrene Copolymer, Hydrogenated Ethylene/Propylene/Styrene Copolymer, Hydrogenated Japan Wax, Hydrogenated Polyisobutene, Hydrogenated Styrene/Butadiene Copolymer, Hydrogenated Styrene/Methyl Styrene/Indene Copolymer, Hydroxypropylcellulose, Isobutylene/Isoprene Copolymer, Lithium Oxidized Polyethylene, Methoxy PEG-17/Dodecyl Glycol Copolymer, Methoxy PEG-22/Dodecyl Glycol Copolymer, Methyl Methacrylate Crosspolymer, Methylstyrene/Vinyltoluene Copolymer, Microcrystalline Wax, Montan Acid Wax, Montan Wax, Myrica Cerifera (Bayberry) Fruit Wax, Nylon-611/Dimethicone Copolymer, Octadecene/MA Copolymer, Oleic/Linoleic/Linolenic Polyglycerides, Ouricury Wax, Oxidized Beeswax, Oxidized Microcrystalline Wax, Oxidized Polyethylene, Oxidized Polypropylene, Ozokerite, Paraffin, PEG-18 Castor Oil Dioleate, PEG-10 Dimethicone Crosspolymer, PEG-12 Dimethicone Crosspolymer, PEG-5 Hydrogenated Castor Oil Isostearate, PEG-10 Hydrogenated Castor Oil Isostearate, PEG-20 Hydrogenated Castor Oil Isostearate, PEG-30 Hydrogenated Castor Oil Isostearate, PEG-40 Hydrogenated Castor Oil Isostearate, PEG-50 Hydrogenated Castor Oil Isostearate, PEG-58 Hydrogenated Castor Oil Isostearate, PEG-50 Hydrogenated Castor Oil Succinate, PEG-5 Hydrogenated Castor Oil Triisostearate, PEG-10 Hydrogenated Castor Oil Triisostearate, PEG-15 Hydrogenated Castor Oil Triisostearate, PEG-20 Hydrogenated Castor Oil Triisostearate, PEG-30 Hydrogenated Castor Oil Triisostearate, PEG-40 Hydrogenated Castor Oil Triisostearate, PEG-60 Hydrogenated Castor Oil Triisostearate, PEG-5 Lanolinamide, PEG-5 Oleamide Dioleate, Phthalic Anhydride/Butyl Benzoic Acid/Propylene Glycol Copolymer, Phthalic Anhydride/Glycerin/Glycidyl Decanoate Copolymer, Phthalic Anhydride/Trimellitic Anhydride/Glycols Copolymer, Piperylene/Butene/Pentene Copolymer, Polybutene, Polybutylene Terephthalate, Polycyclopentadiene, Polydipentene, Polyethylene, Polyethylene Terephthalate, Polyglyceryl-3 Polyricinoleate, Polyglyceryl-4 Polyricinoleate, Polyglyceryl-5 Polyricinoleate, Polyglyceryl-10 Polyricinoleate, Polyisobutene, Polyisoprene, Polypentene, Polyperfluoroethoxymethoxy Difluoromethyl Distearamide, Polypropylene, Polysilicone-4, Polysilicone-5, Polysilicone-17, Polystyrene, Polyvinyl Butyral, Polyvinyl Laurate, Potassium Oxidized Microcrystalline Wax, Potassium PEG-50 Hydrogenated Castor Oil Succinate, PVM/MA Decadiene Crosspolymer, PVP/Decene Copolymer, Rhus Succedanea Fruit Wax, Rosin, Silica Dimethicone Silylate, Silica Dimethyl Silylate, Simmondsia Chinensis (Jojoba) Seed Wax, Sodium PVM/MA/Decadiene Crosspolymer, Spent Grain Wax, Steareth-10 Allyl Ether/Acrylates Copolymer, Steareth-60 Cetyl Ether, Stearoxymethicone/Dimethicone Copolymer, Stearyl Methacrylate/Perfluorooctylethyl Methacrylate Copolymer, Styrene/Methacrylamide/Acrylates Copolymer, Synthetic Beeswax, Synthetic Candelilla Wax, Synthetic Carnauba, Synthetic Japan Wax, Synthetic Wax, TDI Oxidized Microcrystalline Wax, Tricontanyl PVP, Trifluoropropyl Dimethicone Crosspolymer, Trifluoropropyl Dimethicone/Trifluoropropyl Divinyldimethicone Crosspolymer, Trifluoropropyl Dimethicone/Vinyl Trifluoropropyl Dimethicone/Silsesquioxane Crosspolymer, Trimethylpentanediol/Isophthalic Acid/Trimellitic Anhydride Copolymer, Trimethylsiloxysilicate/Dimethiconol Crosspolymer, Vinyl Dimethicone/Lauryl Dimethicone Crosspolymer, Vinyl Dimethicone/Methicone Silsesquioxane Crosspolymer, VP/Eicosene Copolymer, VP/Hexadecene Copolymer.

Es ist erfindungsgemäß bevorzugt, der pulverförmigen Zusammensetzung 0,005 bis 10,0 Gew.-%, besonders bevorzugt von 0,01 bis 3,0 Gew.-% - jeweils bezogen auf das Gewicht des Mittels - Verdicker für die Ölphase zuzufügen.

Insbesondere sollte die Ölphase in der erfindungsgemäß pulverförmigen Zusammensetzung eine solche Menge an Verdickern für die Ölphase enthalten, so dass die Ölphase die als bevorzugt genannte Viskosität (*vide supra*) besitzt.
Ebenso ist es möglich, den Verdicker für die Ölphase der pulverförmigen Zusammensetzung in Form eines Pulvers zu formulieren. Dabei kann die Ölphase gleichzeitig ebenso einen Verdicker für die Ölphase enthalten.

Die verwendete pulverförmige Zusammensetzung kann weiterhin die Hilfs- und Zusatzstoffe enthalten, die üblicherweise herkömmlichen Stylingmitteln zugesetzt werden.

Als geeignete Hilfs- und Zusatzstoffe sind insbesondere Pflegestoffe zu nennen.

Als Pflegestoff kann ein kationisches Tensid eingesetzt werden. Bevorzugt sind dabei kationische Tenside vom Typ der quartären Ammoniumverbindungen, der Esterquats und der Amidoamine. Bevorzugte quartäre Ammoniumverbindungen sind Ammoniumhalogenide, insbesondere Chloride und Bromide, wie Alkyltrimethylammoniumchloride, Dialkyldimethylammoniumchloride und Trialkylmethylammoniumchloride, z. B. Cetyltrimethylammoniumchlorid, Stearyltrimethylammoniumchlorid, Distearyldimethylammoniumchlorid, Lauryldimethylammoniumchlorid, Lauryldimethylbenzylammoniumchlorid und Tricetylmethylammoniumchlorid, sowie die unter den INCI-Bezeichnungen Quaternium-27 und Quaternium-83 bekannten Imidazolium-Verbindungen. Die langen Alkylketten der oben genannten Tenside weisen bevorzugt 10 bis 18 Kohlenstoffatome auf. Da sich der Zusatz oberflächenaktiver Substanzen jedoch negativ auf die hydrophoben Eigenschaften des hydrophobierten Siliciumdioxids und damit auf die Stabilität der verwendeten pulverförmigen Zusammensetzungen auswirken kann, ist die Menge an pflegendem Tensid sorgfältig auf die Gesamtzusammensetzung abzustimmen. Vorzugsweise wird auf den Zusatz tensidischer Bestandteile verzichtet.

Als Pflegestoff eignen sich ebenfalls pflegende Polymere.

Eine erste Gruppe der pflegenden Polymere sind die kationischen Polymere. Unter kationischen Polymeren sind Polymere zu verstehen, welche in der Haupt- und/oder Seitenkette eine Gruppe aufweisen, welche "temporär" oder "permanent" kationisch sein kann. Als "permanent kationisch" werden erfindungsgemäß solche Polymere bezeichnet, die unabhängig vom pH-Wert des Mittels eine kationische Gruppe aufweisen. Dies sind in der Regel Polymere, die ein quartäres Stickstoffatom, beispielsweise in Form einer Ammoniumgruppe, enthalten. Bevorzugte kationische Gruppen sind quartäre Ammoniumgruppen. Insbesondere solche Polymere, bei denen die quartäre Ammoniumgruppe über eine C₁₋₄-Kohlenwasserstoffgruppe an eine aus Acrylsäure, Methacrylsäure oder deren Derivaten aufgebaute Polymerhauptkette gebunden sind, haben sich als besonders geeignet erwiesen.

Ein besonders geeignetes Homopolymer ist das, gewünschtenfalls vernetzte, Poly(methacryloyloxyethyltrimethylammoniumchlorid) mit der INCI-Bezeichnung Polyquaternium-37. Die Vernetzung kann gewünschtenfalls mit Hilfe mehrfach olefinisch ungesättigter Verbindungen, beispielsweise Divinylbenzol, Tetraallyloxyethan, Methylenbisacrylamid, Diallylether, Polyallylpolyglycerylether, oder Allylethern von Zuckern oder Zuckerderivaten wie Erythritol, Pentaerythritol, Arabitol, Mannitol, Sorbitol, Sucrose oder Glucose erfolgen. Methylenbisacrylamid ist ein bevorzugtes Vemetzungsagens.

Weiterhin sind kationisierte Proteinhydrolysate zu den kationischen Polymeren zu zählen, wobei das zugrunde liegende Proteinhydrolysat vom Tier, beispielsweise aus Collagen, Milch oder Keratin, von der Pflanze, beispielsweise aus Weizen, Mais, Reis, Kartoffeln, Soja oder Mandeln, von marinen Lebensformen, beispielsweise aus Fischcollagen oder Algen, oder biotechnologisch gewonnenen Proteinhydrolysaten, stammen kann. Die den erfindungsgemäßen kationischen Derivaten zugrunde liegenden Proteinhydrolysate können aus den entsprechenden Proteinen durch eine chemische, insbesondere alkalische oder saure Hydrolyse, durch eine enzymatische Hydrolyse und/oder eine Kombination aus beiden Hydrolysearten gewonnen werden. Die Hydrolyse von Proteinen ergibt in der Regel ein Proteinhydrolysat mit einer Molekulargewichtsverteilung von etwa 100 Dalton bis hin zu mehreren tausend Dalton. Bevorzugt sind solche kationischen Proteinhydrolysate, deren zugrunde liegender Proteinanteil ein Molekulargewicht von 100 bis zu 25000 Dalton, bevorzugt 250 bis 5000 Dalton aufweist. Weiterhin sind unter kationischen Proteinhydrolysaten quatemierte Aminosäuren und deren Gemische zu verstehen. Die Quaternisierung der Proteinhydrolysate oder der Aminosäuren wird häufig mittels quartären Ammoniumsalzen wie beispielsweise N,N-Dimethyl-N-(n-Alkyl)-N-(2-hydroxy-3-chloro-n-propyl)-ammoniumhalogeniden durchgeführt. Weiterhin können die kationischen Proteinhydrolysate auch noch weiter derivatisiert sein. Als typische Beispiele für die erfindungsgemäßen kationischen Proteinhydrolysate und -derivate seien die unter den INCI - Bezeichnungen im "International Cosmetic Ingredient Dictionary and Handbook", (seventh edition 1997, The Cosmetic, Toiletry, and Fragrance Association 1101 17^{th} Street, N.W., Suite 300, Washington, DC 20036-4702) genannten und im Handel erhältlichen Produkte genannt: Cocodimonium Hydroxypropyl Hydrolyzed Collagen, Cocodimonium Hydroxypropyl Hydrolyzed Casein, Cocodimonium Hydroxypropyl Hydrolyzed Collagen, Cocodimonium Hydroxypropyl Hydrolyzed Hair Keratin, Cocodimonium Hydroxypropyl Hydrolyzed Keratin, Cocodimonium Hydroxypropyl Hydrolyzed Rice Protein, Cocodimonium Hydroxypropyl Hydrolyzed Soy Protein, Cocodimonium Hydroxypropyl Hydrolyzed Wheat Protein, Hydroxypropyl Arginine Lauryl/Myristyl Ether HCl, Hydroxypropyltrimonium Gelatin, Hydroxypropyltrimonium Hydrolyzed Casein, Hydroxypropyltrimonium Hydrolyzed Collagen, Hydroxypropyltrimonium Hydrolyzed Conchiolin Protein, Hydroxypropyltrimonium Hydrolyzed Keratin, Hydroxypropyltrimonium Hydrolyzed Rice Bran Protein, Hydroxypropyltrimonium Hydrolyzed Soy Protein, Hydroxypropyl Hydrolyzed Vegetable Protein, Hydroxypropyltrimonium Hydrolyzed Wheat Protein, Hydroxypropyltrimonium Hydrolyzed Wheat Protein/Siloxysilicate, Laurdimonium Hydroxypropyl Hydrolyzed Soy Protein, Laurdimonium Hydroxypropyl Hydrolyzed Wheat Protein, Laurdimonium Hydroxypropyl Hydrolyzed Wheat Protein/Siloxysilicate, Lauryldimonium Hydroxypropyl Hydrolyzed Casein, Lauryldimonium Hydroxypropyl Hydrolyzed Collagen, Lauryldimonium Hydroxypropyl Hydrolyzed Keratin, Lauryldimonium Hydroxypropyl Hydrolyzed Soy Protein, Steardimonium Hydroxypropyl Hydrolyzed Casein, Steardimonium Hydroxypropyl Hydrolyzed Collagen, Steardimonium Hydroxypropyl Hydrolyzed Keratin, Steardimonium Hydroxypropyl Hydrolyzed Rice Protein, Steardimonium Hydroxypropyl Hydrolyzed Soy Protein, Steardimonium Hydroxypropyl Hydrolyzed Vegetable Protein, Steardimonium Hydroxypropyl Hydrolyzed Wheat Protein, Steartrimonium Hydroxyethyl Hydrolyzed Collagen, Quaternium-76 Hydrolyzed Collagen, Quatemium-79 Hydrolyzed Collagen, Quaternium-79 Hydrolyzed Keratin, Quaternium-79 Hydrolyzed Milk Protein, Quatemium-79 Hydrolyzed Soy Protein, Quaternium-79 Hydrolyzed Wheat Protein.

Bevorzugt sind die kationischen Proteinhydrolysate und -derivate auf pflanzlicher Basis.

Weitere erfindungsgemäß einsetzbare pflegende Polymere sind amphotere Polymere.

Als Pflegestoff kann weiterhin mindestens ein Vitamin, ein Provitamin, eine Vitaminvorstufe und/oder eines derer Derivate eingesetzt werden.

Dabei sind erfindungsgemäß solche Vitamine, Pro-Vitamine und Vitaminvorstufen bevorzugt, die üblicherweise den Gruppen A, B, C, E, F und H zugeordnet werden. Besonders bevorzugt sind Vitamine, die zur B-Gruppe oder zu dem Vitamin B-Komplex gehören, ganz besonders bevorzugt Vitamin B₅ (Pantothensäure, Panthenol und Pantolacton).

Als Pflegestoff kann weiterhin mindestens ein Pflanzenextrakt eingesetzt werden.

Üblicherweise werden diese Extrakte durch Extraktion der gesamten Pflanze hergestellt. Es kann aber in einzelnen Fällen auch bevorzugt sein, die Extrakte ausschließlich aus Blüten und/oder Blättern der Pflanze herzustellen.

Hinsichtlich der erfindungsgemäß bevorzugten Pflanzenextrakte wird insbesondere auf die Extrakte hingewiesen, die in der auf Seite 44 der 3. Auflage des Leitfadens zur Inhaltsstoffdeklaration kosmetischer Mittel, herausgegeben vom Industrieverband Körperpflege- und Waschmittel e.V. (IKW), Frankfurt, beginnenden Tabelle aufgeführt sind.

Erfindungsgemäß sind vor allem die Extrakte aus Seerose, Grünem Tee, Eichenrinde, Brennnessel, Hamamelis, Hopfen, Henna, Kamille, Klettenwurzel, Schachtelhalm, Weißdorn, Lindenblüten, Mandel, Aloe Vera, Fichtennadel, Rosskastanie, Sandelholz, Wacholder, Kokosnuss, Mango, Aprikose, Limone, Weizen, Kiwi, Melone, Orange, Grapefruit, Salbei, Rosmarin, Birke, Malve, Wiesenschaumkraut, Quendel, Schafgarbe, Thymian, Melisse, Hauhechel, Huflattich, Eibisch, Meristem, Ginseng und Ingwerwurzel bevorzugt.

Als Pflegestoff eignen sich weiterhin eine Reihe von Carbonsäuren.

Vorteilhaft im Sinne der Erfindung können insbesondere kurzkettige Carbonsäuren sein. Unter kurzkettigen Carbonsäuren und deren Derivaten im Sinne der Erfindung werden Carbonsäuren verstanden, welche gesättigt oder ungesättigt und/oder geradkettig oder verzweigt oder cyclisch und/oder aromatisch und/oder heterocyclisch sein können und ein Molekulargewicht kleiner 750 aufweisen. Bevorzugt im Sinne der Erfindung können gesättigte oder ungesättigte geradkettige oder verzweigte Carbonsäuren mit einer Kettenlänge von 1 bis zu 16 C-Atomen in der Kette sein, ganz besonders bevorzugt sind solche mit einer Kettenlänge von 1 bis zu 12 C - Atomen in der Kette.

Weitere geeignete Pflegestoffe sind Proteinhydrolysate und/oder deren Derivate, wobei die Verwendung von Proteinhydrolysaten pflanzlichen Ursprungs, z. B. Soja-, Mandel-, Erbsen-, Kartoffel- und Weizenproteinhydrolysaten, bevorzugt ist. Solche Produkte sind beispielsweise unter den Warenzeichen Gluadin^{®} (Cognis), DiaMin^{®} (Diamalt), Lexein^{®} (Inolex), Hydrosoy^{®} (Croda), Hydrolupin^{®} (Croda), Hydrosesame^{®} (Croda), Hydrotritium^{®} (Croda) und Crotein^{®} (Croda) erhältlich.

Wenngleich der Einsatz der Proteinhydrolysate als solche bevorzugt ist, können an deren Stelle gegebenenfalls auch anderweitig erhaltene Aminosäuregemische eingesetzt werden. Ebenfalls möglich ist der Einsatz von Derivaten der Proteinhydrolysate, beispielsweise in Form ihrer Fettsäure-Kondensationsprodukte. Solche Produkte werden beispielsweise unter den Bezeichnungen Lamepon^{®} (Cognis), Lexein^{®} (Inolex), Crolastin^{®} (Croda), Crosilk^{®} (Croda) oder Crotein^{®} (Croda) vertrieben.

Selbstverständlich umfasst die erfindungsgemäße Lehre alle isomeren Formen, wie cis - trans - Isomere, Diastereomere und chirale Isomere.

Erfindungsgemäß ist es auch möglich, eine Mischung aus mehreren Proteinhydrolysaten einzusetzen.

Weiterhin sind als Pflegestoff Lipide und Ölkörper, beispielsweise pflanzliche Öle, flüssige Paraffinöle, Isoparaffinöle, synthetische Kohlenwasserstoffe und Esteröle, Enzyme und Perlenextrakte geeignet.

Neben den Pflegestoffen können auch weitere Hilfs- und Zusatzstoffe zugegeben werden.

Durch Zugabe eines UV-Filters können sowohl die Zubereitungen selbst, als auch die behandelten Fasern vor schädlichen Einflüssen von UV-Strahlung geschützt werden. Es kann daher vorteilhaft sein, den pulverförmigen Zubereitungen mindestens einen UV-Filter zuzugeben. Die geeigneten UV-Filter unterliegen hinsichtlich ihrer Struktur und ihrer physikalischen Eigenschaften keinen generellen Einschränkungen. Vielmehr eignen sich alle im Kosmetikbereich einsetzbaren UV-Filter, deren Absorptionsmaximum im UVA(315-400 nm)-, im UVB(280-315nm)- oder im UVC(<280 nm)-Bereich liegt. UV-Filter mit einem Absorptionsmaximum im UVB-Bereich, insbesondere im Bereich von etwa 280 bis etwa 300 nm, sind besonders bevorzugt.

Die erfindungsgemäß bevorzugten UV-Filter können beispielsweise ausgewählt werden aus substituierten Benzophenonen, p-Aminobenzoesäureestern, Diphenylacrylsäureestern, Zimtsäureestern, Salicylsäureestern, Benzimidazolen und o-Aminobenzoesäureestem. Beispielhaft sei hier 2-Hydroxy-4-methoxybenzophenon-5-sulfonsäure und deren Natriumsalz (Benzophenone-4; Uvinul^{®}MS 40; Uvasorb^{®}S 5) genannt.

In einer besonderen Ausführungsform enthält die eingesetzte pulverförmige Zusammensetzung weiterhin einen oder mehrere direktziehende Farbstoffe. Dies ermöglicht, dass bei Anwendung des Mittels die behandelte keratinische Faser nicht nur temporär strukturiert, sondern zugleich auch gefärbt wird. Das kann insbesondere dann wünschenswert sein, wenn nur eine temporäre Färbung beispielsweise mit auffälligen Modefarben gewünscht wird, die sich durch einfaches Waschen wieder aus der keratinischen Faser entfernen lässt.

Auch die Zugabe eines Tensids ist nicht ausgeschlossen, jedoch wegen der bereits erwähnten nachteiligen Beeinflussung der Hydrophobizität des Metalloxids und damit der Stabilität der pulverförmigen Zusammensetzung nicht bevorzugt.

Auch die übliche Zugabe von Parfümkomponenten und Konservierungsmitteln ist möglich.

Weiterhin können die pulverförmigen Zusammensetzungen Alkalisierungsmittel, üblicherweise Alkali- oder Erdalkalihydroxide, Ammoniak oder organische Amine, enthalten. Bevorzugte Alkalisierungsmittel sind Monoethanolamin, Monoisopropanolamin, 2-Amino-2-methyl-propanol, 2-Amino-2-methyl-1,3-propandiol, 2-Amino-2-ethyl-1,3-propandiol, 2-Amino-2-methylbutanol und Triethanolamin sowie Alkali- und Erdalkalimetallhydroxide. Insbesondere Monoethanolamin, Triethanolamin sowie 2-Amino-2-methyl-propanol und 2-Amino-2-methyl-1,3-propandiol sind im Rahmen dieser Gruppe bevorzugt. Auch die Verwendung von ω-Aminosäuren wie ω-Aminocapronsäure als Alkalisierungsmittel ist möglich.

Die pulverförmigen Zusammensetzungen, die erfindungsgemäß zur temporären Verformung keratinischer Fasern eingesetzt werden, lassen sich einfach herstellen. Es hat sich bewährt, zunächst auf bekannte Weise, etwa durch einfaches Verrühren, eine Ölphase - insbesondere eine Dispersion des filmbildenden und/oder festigenden Polymers und der gewünschten Hilfs- und Zusatzstoffe in der Ölphase - herzustellen, diese in einem Mischer vorzulegen und schließlich das hydrophobierte Metalloxidpulver unter intensivem Rühren zuzugeben. Die benötigte Mischzeit ist abhängig von der eingebrachten Mischenergie und der jeweiligen Zusammensetzung der Mischung, beträgt aber in der Regel zwischen 15 Sekunden und 5 Minuten. Wird zu kurz gemischt, bildet sich kein stabiles Pulver aus und es kommt zur Ausbildung einer öligen Phase. Bei zu langer Mischzeit wird das zunächst entstehende Pulver in eine brei- oder cremeartige Konsistenz überführt, wobei dieser Vorgang nicht reversibel verläuft. Es empfiehlt sich daher, durch einige Vorversuche die optimale Mischzeit für das jeweilige System zu ermitteln.

Die pulverförmigen Zusammensetzungen können in nahezu beliebigen Behältnissen konfektioniert werden. Es muss lediglich sichergestellt werden, dass die mechanische Belastung des Pulvers bei der Entnahme der Zusammensetzung nicht so hoch ist, dass bereits bei der Entnahme das Pulver in flüssige Form überführt wird. Geeignet sind beispielsweise Tiegel, Flaschen oder auch Tetrapacks, wobei das Behältnis beispielsweise mit einer Schütt- und Dosiervorrichtung ausgestaltet werden kann. Bei der Verwendung der pulverförmigen Zusammensetzung zur temporären Verformung keratinischer Fasern wird zunächst die gewünschte Menge der pulverförmigen Zusammensetzung dem Behältnis entnommen. Die Zusammensetzung kann dabei direkt auf die zu behandelnde keratinische Faser oder aber beispielsweise auf die Hand gegeben werden. Im ersten Fall kann das aufgebrachte Pulver direkt auf der keratinischen Faser einer mechanischen Belastung, beispielsweise mittels der Hände ausgesetzt werden, wodurch das Lösungsmittel und das filmbildende und/oder festigende Polymer direkt auf der Faser freigesetzt werden. Wird die pulverförmige Zusammensetzung zunächst auf die Hand gegeben, so kann es zunächst vorsichtig im Haar verteilt und wiederum erst anschließend stärker mechanisch belastet werden, beispielsweise durch gezieltes Einmassieren des Pulvers in das Haar. Dadurch wird das haltgebende Polymer erst direkt auf der gewünschten Haarpartie freigesetzt. So lässt sich sehr gezielt eine hervorragende Stylingwirkung erreichen. Es ist natürlich auch möglich, die pulverförmige Zusammensetzung bereits auf der Hand zu verreiben und erst das entstehende flüssige oder pastenartige Mittel auf die keratinische Faser aufzubringen. Diese Vorgehensweise ist allerdings nicht bevorzugt, da dabei auf einen wesentlichen Vorteil der pulverförmigen Konsistenz des Stylingmittels, nämlich die gute Verteilbarkeit, verzichtet wird. Die pulverförmige Zusammensetzung lässt sich natürlich auch mit einem Hilfsmittel, etwa einem Pinsel, einem Schwamm, einem Tuch, einer Bürste oder einem Kamm auftragen.

Ein zweiter Gegenstand der Erfindung ist die Verwendung einer pulverförmigen Zusammensetzung des ersten Erfindungsgegenstandes zur Erzeugung von Glanz auf keratinischen Fasern, insbesondere menschlichen Haaren.

Ein dritter Gegenstand der Erfindung ist die Verwendung einer pulverförmigen Zusammensetzung des ersten Erfindungsgegenstandes zur temporären Umformung keratinischer Fasern, insbesondere menschlicher Haare.

Ein vierter Gegenstand der Erfindung ist ein Verfahren zur temporären Umformung und/oder Glanzgebung keratinischer Fasern, insbesondere menschlicher Haare, worin eine pulverförmige Zusammensetzung des ersten Erfindungsgegenstandes vor oder während der Applikation auf die keratinischen Fasern mechanisch, insbesondere durch Druck oder Reibung, unter Freisetzung einer flüssigen Zusammensetzung beansprucht wird und die aus der mechanischen Beanspruchung der pulverförmigen Zusammensetzung entstandene flüssige Zusammensetzung auf die keratinischen Fasern einwirkt.

Es hat sich als bevorzugt herausgestellt, wenn die keratinischen Fasern nach der Einwirkung der aus der pulverförmigen Zusammensetzung des ersten Erfindungsgegenstandes resultierenden flüssigen Zusammensetzung nicht gespült werden.

Die nachfolgenden Beispiele sollen den Gegenstand der vorliegenden Erfindung erläutern ohne ihn in irgendeiner Weise zu beschränken.

### Beispiele

Folgende Creme-in-Pulver-Formulierung wurde als glanzbringendes Haarstylingpulver durch mischen bereitgestellt:

| | |
|---|---|
| Titaniumdioxide (and) Perfluoroalkylphosphate (PW Covafluor, LCW) | 28.00 g |
| Talc (and) perfluoralkylphosphate (PFS Talc JA 46-R, Daito) | 8.50 g |
| Mica (Submica M) | 8.50 g |
| Amphomer^{® 1} | 1.00 g |
| Methylmethacrylate Crosspolymer (Covabead LH 170, LCW) | 3.50 g |
| Hydrogenated Polyisobutene (Squatol S, LCW) | 50.50 g |

| | |
|---|---|
| Amphomer^{®} INCI-Bezeichnung: Octylacrylamide/Acrylates/Butyl-aminoethyl Methacrylate Copolymer (National Starch) | |

## Patentansprüche

1. Pulverformige Zusammensetzung zur temporären Verförmung keratinischer Fasern, enthaltend
- 0 bis 20 Gew.-% Wasser,
- als Ölphase 40 Gew.-% bis 95 Gew.-% mindestens eines Öls,
- mindestens ein partikeltörmiges, hydrophobiertes Metalloxid,
- zusätzlich mindestens ein filmbildendes und/oder festlgendes Polymer.

2. Pulverförmige Zusammensetzung nach Anspruch 1, **dadurch gekennzeichnet, dass** das Öl ausgewählt wird aus mindestens einem Öl der Gruppe, die gebildet wird aus pflanzlichen Ölen,
flüssigen Paraffinölen,
Isoparaffinölen,
flüssigen synthetischen Kohlenwasserstoffen,
flüssigen Di-n-alkylethern,
Esterölen,
flüssigen Silikonen,
Dicarbonsäureestern,
symmetrischen, unsymmetrischen oder cyclischen Estern der Kohlensäure, Mono,- Di- und Trifettsäureester von gasättiglen und/oder ungesättigten linearen und/oder verzweigten Fettsäuren mit Glycerin.

3. Pulverförmige Zusammensetzung nach einem der Ansprüche 1 oder 2, **dadurch gekennzeichnet, dass** die Ölphase zusätzlich mindestens einen Fettstoff enthält.

4. Pulverförmige Zusammensetzung nach einem der Ansprüche 1 bis 3, **dadurch gekennzeichnet, dass** partikelförmige, hydrophobierte Metalloxide in einer Menge von 0,5 Gew.-% bis 15 Gew.-% enthalten sind.

5. Pulverförmige Zusammensetzung nach einem der Ansprüche 1 bis 4, **dadurch gekennzeichnet, dass** die partikelförmigen, hydrophobierten Metalloxide einen Partikeldurchmesser von weniger als 5 µm, bevorzugt weniger als 1 µm, besonders bevorzugt zwischen 20 und 100 nm, aufweisen.

6. Pulverförmige Zusammensetzung nach einem der Ansprüche 1 bis 5, **dadurch gekennzeichnet, dass** das partikelförrrige, hydrophobierte Metalloxid ausgewählt wird aus partikelförmigem, hydrophobiertem Siliziumdioxid und/oder partikelförmigem, hydrophobiertem Titandioxid und/oder partikelförmigem, hydrophobiertem Aluminiumoxid (Al₂O₃) und/oder partikelförmigem, hydrophobiertem Phyllosilikat.

7. Pulverförmige Zusammensetzung nach einem der Ansprüche 1 bis 6, **dadurch gekennzeichnet, dass** das partikelformige, hydrophobierte Metalloxid an der Oberfläche mit mindestens einem Rest hydrophobiert wurde, ausgewähit aus Trialkylsilyl, Perfluoralkylsilyl, Polyperfluoralkylenoxid, Perfluoralkylpolyalkylenoxid, Polysiloxan mit Perfluoralkylgruppen, Perfluoralkylphosphat, Polyfluoralkylphosphatether.

8. Pulverförmige Zusammensetzung nach Anspruch 1 bis 7, **dadurch gekennzeichnet, dass** als filmbildendes und/oder festigendes Polymer mindestens ein filmbildendes und/oder festigendes Polymer enthalten ist, das aus mindestens einem folgenden Monomere aufgebaut ist Vinylpyrrolidon, Vinylcaprolactam, Vinylester, Vinylalkohol, Acrylamid, Methacrylamid, C₁- bis C₇-Alkylacrylamid, C₁- bis C₇-Dielkylacrylamid, C₁- bis C₇-Alkyltrethacrylamid, C₁- bis C₇-Dialkylmethacrylamid, C₁- bis C₇-Alkylacrylat, Acrylsäure, Propylen-glykol, Ethylenglykol.

9. Pulverförmige Zusammensetzungen nach einem der Ansprüche 1 bis 8, **dadurch gekennzeichnet, dass** die filmbildenden und/oder festigenden Polymere ausgewählt werden aus der Gruppe, die gebildet wird aus Vinylpyrrolidon-Vinylacetat-Copolymeren, Vinylcetat-Crotonsäure-Copolymeren, Vinylacetat-Butylmaleat-Isobornylacrylat-Copolymeren, Octylacrylamid-Acrylat-Butylaminoethylmethacrylat-Copolymeren und quaternierten Vinylpyrrolidon-Dimethylaminoethylmelhacrylat-Copolymeren.

10. Pulverförmig Zusammensetzung nach einem der Ansprüche 1 bis 9, dadurch gekennzelchnet, dass die filmbildenden und/oder festigenden Polymere In der Ölphase dispergiert oder gelöst vorliegen.

11. Pulverförmige Zusammensetzung nach einem der Ansprüche 1 bis 10, **dadurch gekennzeichnet, dass** sie zusätzlich mindestens einen Verdicker für die Ölphase enthält.

12. Verwendung einer pulverförmigen Zusammensetzung nach einem der Ansprüche 1 bis 11 zur Erzeugung von Glanz auf keratinischen Fasern, insbesondere auf menschlichem Haar.

13. Verwendung einer pulverförmigen Zusammensetzung nach einem der Ansprüche 1 bis 11 zur temporären Umformung keratinischer Fasern, insbesondere menschlicher Haare.

14. Verfahren zur temporären Umformung und/oder Glanzgebung keratinischer Fasern, Insbesondere menschlicher Haare, **dadurch gekennzeichnet, dass** eine pulverförmige Zusammensetzung nach einem der Ansprüche 1 bis 11 vor oder während der Applikation auf die keratinischen Fasern mechanisch, insbesondere durch Druck oder Reibung, unter Freisetzung einer flüssigen Zusammensetzung beansprucht wird und die aus der mechanischen Beanspruchung der pulverförmigen Zusammensetzung entstandene flüssige Zusammensetzung auf die keratinischen Fasern einwirkt.

## Claims

1. Pulverulent composition for temporarily shaping keratinic fibres comprising
- 0 to 20 wt % of water,
- as the oil phase 40 wt % to 95 wt % of at least one oil,
- at least one particulate, hydrophobised metal oxide,
- in addition, at least one film-forming and/or setting polymer.

2. Pulverulent composition according to claim 1, **characterised in that** the oil is selected from at least one oil of the group that consists of
vegetal oils,
liquid paraffinic oils,
isoparaffinic oils,
liquid synthetic hydrocarbons,
liquid di-n-alkyl ethers,
ester oils,
liquid silicones,
dicarboxylic acid esters,
symmetrical, unsymmetrical or cyclic esters of carbonic acid,
mono, di and trifatty acid esters of saturated and/or unsaturated linear and/or branched fatty acids with glycerine.

3. Pulverulent composition according to one of claims 1 or 2, characterised - in that the oil phase additionally comprises at least one fatty substance.

4. Pulverulent composition according to one of claims 1 to 3, **characterised in that** particulate, hydrophobised metal oxides are comprised in an amount of 0.5 wt % to 15 wt %.

5. Pulverulent composition according to one of claims 1 to 4, **characterised in that** the particle diameter of the particulate, hydrophobised metal oxides is less than 5 µm, preferably less than 1 µm, particularly preferably between 20 and 100 nm.

6. Pulverulent composition according to one of claims 1 to 5, **characterised in that** the particulate, hydrophobised metal oxide is selected from particulate, hydrophobised silicon dioxide and/or particulate, hydrophobised titanium dioxide and/or particulate, hydrophobised aluminium oxide (Al₂O₃) and/or particulate, hydrophobised phyllosilicate.

7. Pulverulent composition according to one of claims 1 to 6, **characterised in that** the particulate, hydrophobised metal oxide was hydrophobised on the surface with at least one group, selected from trialkylsilyl, perfluoroalkylsilyl, polyperfluoroalkylene oxide, perfluoroalkylpolyalkylene oxide, polysiloxane with perfluoroalkyl groups, perfluoroalkyl phosphate, polyfluoroalkyl phosphate ether.

8. Pulverulent composition according to claim 1 to 7, **characterised in that** at least one film-forming and/or setting polymer is comprised as the film-forming or setting polymer and is formed from at least one of the following monomers: vinyl pyrrolidone, vinyl caprolactam, vinyl esters, vinyl alcohol, acrylamide, methacrylamide, C₁- to C₇ alkylacrylamide, C₁-to C₇ dialkylacrylamide, C₁- to C₇ alkylmethacrylamide, C₁- to C₇ dialkylmethacrylamide, C₁- to C₇ alkyl acrylate, acrylic acid, propylene glycol, ethylene glycol.

9. Pulverulent composition according to one of claims 1 to 8, **characterised in that** the film-forming and/or setting polymers are selected from the group that consists of vinyl pyrrolidone-vinyl acetate copolymers, vinyl acetate-crotonic acid copolymers, vinyl acetate-butyl maleate-isobornyl acrylate copolymers, octylacrylamide-acrylate-butylaminoethyl methacrylate copolymers and quaternised vinyl pyrrolidone-dimethylaminoethyl methacrylate copolymers.

10. Pulverulent composition according to one of claims 1 to 9, **characterised in that** the film-forming and/or setting polymers are dispersed or dissolved in the oil phase.

11. Pulverulent composition according to one of claims 1 to 10, **characterised in that** it additionally comprises at least one thickener for the oil phase.

12. Use of a pulverulent composition according to one of claims 1 to 11 for providing sheen to keratinic fibres, in particular to human hair.

13. Use of a pulverulent composition according to one of claims 1 to 11 for the temporary shaping of keratinic fibres, in particular human hair.

14. Method for temporarily shaping and/or providing sheen to keratinic fibres, in particular human hair, **characterised in that** before or during application onto the keratinic fibres a pulverulent composition according to one of claims 1 to 11 is subjected to stress, in particular by pressure or friction, thereby releasing a liquid composition, and the liquid composition resulting from the mechanical stressing of the pulverulent composition acts on the keratinic fibres.

## Revendications

1. Composition pulvérulente pour la permanente temporaire de fibres kératiniques, contenant
- de 0 à 20 % en poids d'eau ;
- à titre de phase huileuse, de 40 % en poids à 95 % en poids d'au moins une huile ;
- au moins un oxyde métallique rendu hydrophobe, sous forme particulaire ;
- en outre, au moins un polymère filmogène et/ou de renforcement.

2. Composition pulvérulente selon la revendication 1, **caractérisée en ce que** l'huile est choisie parmi au moins une huile du groupe qui est formé par
des huiles végétales ;
des huiles paraffiniques liquides ;
des huiles isoparaffiniques ;
des hydrocarbures synthétiques liquides ;
des éthers di-n-alkyliques liquides ;
des huiles esters ;
des silicones liquides ;
des esters d'acides dicarboxyliques ;
des esters symétriques, asymétriques ou cycliques de l'acide carbonique ;
des esters d'acides monogras, digras et trigras d'acides gras linéaires et/ou ramifiés, saturés et/ou insaturés avec du glycérol.

3. Composition pulvérulente selon l'une quelconque des revendications 1 ou 2, **caractérisée en ce que** la phase huileuse contient en outre au moins une substance grasse.

4. Composition pulvérulente selon l'une quelconque des revendications 1 à 3, **caractérisée en ce que** les oxydes métalliques sous forme particulaire, rendus hydrophobes sont contenus en une quantité de 0,5 % en poids à 15 % en poids.

5. Composition pulvérulente selon l'une quelconque des revendications 1 à 4, **caractérisée en ce que** les oxydes métalliques sous forme particulaire, rendus hydrophobes présentent un diamètre de particule inférieur à 5 µm, de préférence inférieur à 1 µm, de manière particulièrement préférée entre 20 et 100 nm.

6. Composition pulvérulente selon l'une quelconque des revendications 1 à 5, **caractérisée en ce que** l'oxyde métallique sous forme particulaire, rendu hydrophobe est choisi parmi du dioxyde de silicium sous forme particulaire, rendu hydrophobe et/ou du dioxyde de titane sous forme particulaire, rendu hydrophobe et/ou de l'oxyde d'aluminium (Al₂O₃) sous forme particulaire, rendu hydrophobe et/ou du phyllosilicate sous forme particulaire, rendu hydrophobe.

7. Composition pulvérulente selon l'une quelconque des revendications 1 à 6, **caractérisée en ce que** l'oxyde métallique sous forme particulaire, rendu hydrophobe a été rendu hydrophobe à la surface avec au moins un résidu choisi parmi un trialkylsilyle, un perfluoroalkylsilyle, un polyoxyde de perfluoroalkylène, un oxyde de perfluoroalkylpolyalkylène, un polysiloxane contenant des groupes perfluoroalkyle, un phosphate de perfluoroalkyle, un polyéther-phosphate de fluoroalkyle.

8. Composition pulvérulente selon l'une quelconque des revendications 1 à 7, **caractérisée en ce qu'**elle contient, à titre de polymère filmogène et/ou de renforcement, au moins un polymère filmogène et/ou de renforcement qui est constitué par au moins un des monomères ci-après, à savoir la vinylpyrrolidone, le vinylcaprolactame, un ester vinylique, l'alcool vinylique, l'acrylamide, le méthacrylamide, un alkyl(en C₁-C₇)acrylamide, un dialkyl(en C₁-C₇)acrylamide, un alkyl(en C₁-C₇)méthacrylamide, un dialkyl(en C₁-C₇)méthacrylamide, un acrylate d'alkyle en C₁-C₇, l'acide acrylique, le propylèneglycol, l'éthylèneglycol.

9. Composition pulvérulente selon l'une quelconque des revendications 1 à 8, **caractérisée en ce que** les polymères filmogènes et/ou de renforcement sont choisis parmi le groupe qui est formé par des copolymères de vinylpyrrolidone-acétate de vinyle, des copolymères d'acétate de vinyle-acide crotonique, des copolymères d'acétate de vinyle-maléate de butyle-acrylate d'isobornyle, des copolymères d'octylacrylamide-acrylate-méthacrylate de butylaminoéthyle et des copolymères quaternisés de vinylpyrrolidone-méthacrylate de diméthylaminoéthyle.

10. Composition pulvérulente selon l'une quelconque des revendications 1 à 9, **caractérisée en ce que** les polymères filmogènes et/ou de renforcement sont présents à l'état dispersé ou dissous dans la phase huileuse.

11. Composition pulvérulente selon l'une quelconque des revendications 1 à 10, **caractérisée en ce qu'**elle contient en outre au moins un épaississant pour la phase huileuse.

12. Utilisation d'une composition pulvérulente selon l'une quelconque des revendications 1 à 11, pour l'obtention de brillant sur des fibres kératiniques, en particulier sur des cheveux humains.

13. Utilisation d'une composition pulvérulente selon l'une quelconque des revendications 1 à 11, pour la permanente temporaire de fibres kératiniques, en particulier de cheveux humains.

14. Procédé pour la permanente temporaire et/ou le lustrage de fibres kératiniques, en particulier de cheveux humains, **caractérisé en ce qu'**on soumet une composition pulvérulente selon l'une quelconque des revendications 1 à 11, avant ou pendant l'application sur les fibres kératiniques, à une sollicitation mécanique, en particulier par pression ou par friction, avec libération d'une composition liquide, et on laisse agir sur les fibres kératiniques, la composition liquide que l'on obtient à partir de la sollicitation mécanique de la composition pulvérulente.
